(19) Europäisches Patentamt
European Patent Office
Office européen des brevets

(11) **EP 2 176 301 B1**

(12) **EUROPÄISCHE PATENTSCHRIFT**

(45) Veröffentlichungstag und Bekanntmachung des Hinweises auf die Patenterteilung:
**15.05.2013 Patentblatt 2013/20**

(21) Anmeldenummer: **08786773.5**

(22) Anmeldetag: **01.08.2008**

(51) Int Cl.:
*C08F 2/12* (2006.01)    *C08F 220/34* (2006.01)
*C09D 5/02* (2006.01)    *C09D 133/14* (2006.01)
*C09J 133/14* (2006.01)    *A61K 47/00* (2006.01)
*A23L 1/00* (2006.01)

(86) Internationale Anmeldenummer:
**PCT/EP2008/060154**

(87) Internationale Veröffentlichungsnummer:
**WO 2009/016258 (05.02.2009 Gazette 2009/06)**

(54) **WÄSSRIGE POLYMERDISPERSION AUF BASIS VON N, N-DIETHYLAMINOETHYLMETHACRYLAT, DEREN HERSTELLUNG UND VERWENDUNG**

AQUEOUS POLYMER DISPERSION BASED ON N,N-DIETHYLAMINOETHYL METHACRYLATE, ITS PREPARATION AND USE

DISPERSION POLYMÈRE AQUEUSE À BASE DE N,N-DIÉTHYLAMINOÉTHYLMÉTHACRYLATE, MODE DE PRODUCTION ET UTILISATION CORRESPONDANTS

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **02.08.2007 EP 07113737**

(43) Veröffentlichungstag der Anmeldung:
**21.04.2010 Patentblatt 2010/16**

(73) Patentinhaber: **BASF SE**
**67056 Ludwigshafen (DE)**

(72) Erfinder:
• **ANGEL, Maximilian**
**67105 Schifferstadt (DE)**
• **KOLTER, Karl**
**67117 Limburgerhof (DE)**
• **VOSS, Hartwig**
**67227 Frankenthal (DE)**

(56) Entgegenhaltungen:
**EP-A- 0 172 579    US-A- 4 378 445**

• **DATABASE WPI Week 197846 Thomson Scientific, London, GB; AN 1978-83466A XP002506199 & JP 50 161580 A (TORAY IND INC) 27. Dezember 1975 (1975-12-27)**

**Beschreibung**

Die vorliegende Erfindung betrifft ein Verfahren zur Herstellung einer wässrigen Polymerdispersion durch radikalische Emulsionspolymerisation eines Monomergemischs, das N,N-Diethylaminoethylmethacrylat enthält, die nach diesem Verfahren erhältliche Polymerdispersion und deren Verwendung.

Die DE-AS 1090381 beschreibt ein Verfahren zum Überziehen von Arzneiformen mit im Magen löslichen Dragiermassen. Diese enthalten ein Copolymer aus 20 bis 80 % wenigstens eines Aminoesters der (Meth)acrylsäure und 80 bis 20 % eines Monomers, das als Homopolymer ein wasserunlösliches Polymerisat bildet. Als konkrete Beispiele für geeignete polymerisierbare Aminoester werden die Ester der Acrylsäure und (Meth)acrylsäure mit N,N-Dimethylaminoethanol, N,N-Diethylaminoethanol, N,N-Dimethylaminopropanol und N-(hydroxyethyl)morpholin genannt. Als geeignete Comonomere werden niedere Ester der Acrylsäure und vorzugsweise der (Meth)acrylsäure, wie Acrylsäureethylester, (Meth)acrylsäuremethyl-, -butyl- und -hexylester genannt. Die Herstellung erfolgt durch Lösungspolymerisation in einem organischen Lösungsmittel; ein Ausführungsbeispiel ist nicht angegeben.

Die DE-AS 1219175 beschreibt ein Verfahren zur Herstellung von veterinärmedizinischen Wirkstoffzubereitungen, die gegen die Einwirkung von Pansensäften von Wiederkäuern geschützt sind. Dazu werden diese Zubereitungen mit Copolymeren überzogen, die N,N-Dialkylaminoalkyl(meth)acrylamide und ein Comonomer einpolymerisiert enthalten, das ausgewählt ist unter (Meth)acrylaten, Acrylnitril und Vinylaromaten. Copolymere auf Basis von N,N-Dialkylaminoalkyl(meth)acrylaten werden nach der Lehre dieses Dokuments als nachteilig angesehen, da die Estergruppe im Vergleich zur Amidgruppe im basischen Milieu eher verseift.

Die DE-OS 2135073 beschreibt Überzugsmittel für Arzneiformen, die eine wässrige Polymerdispersion enthalten, wobei das Polymer zu 10 bis 55 Gew.-% aus Monomeren mit einer Carboxylgruppe und/oder einer Monoalkyl- oder Dialkylaminoalkylestergruppe aufgebaut ist. Als geeignetes Monomer wird neben einer Vielzahl weiterer auch Diethylaminoethylmethacrylat (DEAEMA) genannt. Als geeignete Comonomere werden die niederen Ester der (Meth)acrylsäure, vorzugsweise Methylmethacrylat, (Meth)acrylnitril, Vinylaromaten, Vinylchlorid und Vinylacetat genannt. Die Herstellung erfolgt durch wässrige Emulsionspolymerisation, vorzugsweise nach dem Emulsionszulaufverfahren. Konkrete Emulsionspolymerisate auf Basis von DEAEMA sind nicht offenbart.

Die DE-AS 2512238 lehrt zur Bereitstellung von Bindemitteln für Arzneimittelüberzüge mit geringem Restmonomerengehalt die Verwendung eines durch Sprühtrocknen einer Polymerdispersion erhaltenen Pulvers zur Herstellung von Überzugslösungen für diese Arzneiformen. Bezüglich der zur Sprühtrocknung eingesetzten Dispersionen wird auf die DE 1090381, DE 1219175 und DE 2135073 Bezug genommen.

Die DE-OS 2838278 beschreibt Beschichtungen für orale Darreichungsformen für Wiederkäuer aus

a) mindestens einem filmbildenden Polymer mit mindestens einer basischen Aminogruppe und mit einem Stickstoffgehalt von 3 bis 14 %, das in wässrigem Pansenmedium bei einem pH-Wert von über 5,5 innerhalb von 24 Stunden löslich ist, und

b) mindestens einer hydrophoben, in dem Polymer dispergierten Substanz, die ausgewählt ist unter $C_{12}$-$C_{32}$-Fettsäuren, Al-Salzen dieser Fettsäuren und/oder Polycarbonsäuren.

Zur Herstellung der Beschichtung wird eine Lösung in einem organischen Lösungsmittel eingesetzt. Als geeignete Polymere wird eine Vielzahl stickstoffhaltiger Homo- und Copolymere aufgeführt, ohne auf geeignete Verfahren zu deren Herstellung einzugehen. Als Ausführungsbeispiel 29 wird dabei ein Copolymer aus 40 % N,N-Diethylaminoethylmethacrylat aufgeführt, ohne jedoch ein Verfahren zu seiner Herstellung anzugeben.

Die GB 1324087 beschreibt Überzugspolymere für orale Darreichungsformen für Wiederkäuer, die

a) wenigstens ein N,N-Dialkylaminoalkyl(meth)acrylat und

b) wenigstens eine ethylenisch ungesättigte Verbindung, die ausgewählt ist unter Vinylaromaten und deren Derivaten, Vinylestern, Estern der (Meth)acrylsäure und Acrylnitril einpolymerisiert enthalten.

Als geeignete Monomere a) werden N,N-Dimethylaminoethylmethacrylat (DMAEMA) und tert.-Butylaminoethylmethacrylat (TBAEMA) offenbart. Als Comonomer b) wird insbesondere Methylmethacrylat als ungeeignet angesehen, da es zur Bildung von zu brüchigen Überzügen neigt. Als geeignete Polymerisationsverfahren werden Substanz-, Suspensions-, Lösungs- und Emulsionspolymerisation angegeben. Die Copolymere der Ausführungsbeispiele wurden durch Lösungspolymerisation hergestellt.

Die DE 3426587 A1 beschreibt ein Verfahren zum Überziehen von Arzneiformen durch Aufbringen eines Films aus einem flüssigen, filmbildenden Überzugsmittel, welches ein gelöstes Polymerisat mit seitenständigen tertiären Ammoniumsalzgruppen enthält. Zur Herstellung dieser Polymerlösungen können unter anderem Copolymere auf Basis von N,N-Dialkylaminoalkyl(meth)acrylaten mit wässrigen anorganischen oder organischen Säuren in wässrige Ammoniumsalzlösungen überführt werden.

Die DE 3049179 A1 ist eine Zusatzanmeldung zur DE 2512238 und betrifft die Verwendung eines durch Sprühtrocknen nach der Lehre des letztgenannten Dokuments gewonnenen Pulvers in Form einer wässrigen Suspension, die zusätzlich ein Weichmachungsmittel enthält, zur Herstellung von Überzügen durch Thermogelierung.

Die EP 0058765 A2 beschreibt in Magensaft lösliche oder quellbare Überzugsmassen für Arzneiformen, die als Bindemittel ein Emulsionspolymerisat auf Basis von N,N-Dialkylaminoalkyl(meth)acrylaten enthalten, wobei zwischen der Aminogruppe und der (Meth)acrylatgruppe sich eine verzweigte Alkylen- oder Aralkylengruppe mit wenigstens drei in gerader Kette angeordneten Kohlenstoffatomen befindet.

Die WO 2005/055986 und WO 2005/056619 beschreiben Polymere mit vom pH-Wert abhängigem Quell-/Lösungsverhalten und deren Einsatz in Arzneiformen.

Die WO 00/05307 beschäftigt sich mit der Bereitstellung von Überzugs- und Bindemitteln für Arzneiformen, die (Meth)acrylat-Copolymere enthalten, die Monomerreste mit tertiären Aminogruppen aufweisen, wobei eine einfache trockene oder wässrige Weiterverarbeitung möglich sein soll. Dazu lehrt dieses Dokument ein Verfahren, bei dem man (a) ein Copolymer aus $C_1$-$C_4$-Estern der (Meth)acrylsäure und (Meth)acrylatMonomeren, die tertiäre Ammoniumgruppen aufweisen, (b) einen Weichmacher und (c) einen Emulgator mit einem HLB-Wert von mindestens 14 miteinander vermengt und daraus das Überzugs- oder Bindemittel durch Schmelzen, Gießen, Ausstreichen oder Aufsprühen herstellt, wobei das Copolymer (a) in Pulverform mit einer mittleren Teilchengröße von 1 bis 40 $\mu$m eingebracht wird. Die dabei erzielte Verarbeitbarkeit wird der Bereitstellung des Copolymeren (a) in Pulverform mit extrem geringer Korngröße zugeschrieben.

Die WO 02/067906 betrifft Überzugs- und Bindemittel mit verbesserter Wasserdampfdurchlässigkeit gegenüber den in der WO 00/05307 beschriebenen. Dabei erfolgt die Herstellung der Überzugs- und Bindemittel mit einer Mischung, die (a) ein Copolymer aus $C_1$-$C_4$-Estern der (Meth)acrylsäure und weitere (Meth)acrylat-Monomere mit funktionellen tertiären Ammoniumgruppen in Pulverform mit einer mittleren Teilchengröße von 1 bis 40 $\mu$m, (b) einen Emulgator mit einem HLB-Wert von mindestens 14 und (c) eine $C_{12}$-$C_{18}$-Monocarbonsäure oder eine $C_{12}$-$C_{18}$-Hydroxylverbindung enthält.

Die WO 2004/019918 beschreibt Überzugs- und Bindemittel, die bezüglich ihrer Zusammensetzung den in der WO 00/05307 und WO 02/067906 beschriebenen entsprechen.

Der vorliegenden Erfindung liegt die Aufgabe zu Grunde, Polymere und ein Verfahren zu ihrer Herstellung bereitzustellen, die sich als Überzugsmittel für pharmazeutische Darreichungsformen eignen und möglichst einfach herstellen und/oder formulieren lassen. Die Polymere sollen dabei ein Eigenschaftsspektrum aufweisen, das möglichst viele der folgenden Bedingungen gut erfüllt: Überzüge für Arzneiformen auf Basis von Filmen dieser Polymere sollen pH-abhängige Löslichkeitseigenschaften aufweisen, sich durch gute Barriereeigenschaften für Wasserdampf auszeichnen und/oder zur Maskierung schlecht schmeckender Wirkstoffe eignen; die Herstellung soll möglichst einfach gelingen; bei der Formulierung soll auf aufwendige Trocknungs- und/oder andere Konfektionierungsschritte möglichst verzichtet werden.

Überraschenderweise wurde nun gefunden, dass sich Polymerdispersionen, die N,N-Diethylaminoethylmethacrylat (DEAEMA) einpolymerisiert enthalten, durch ein sehr gutes Eigenschaftsprofil auszeichnen. Insbesondere lassen sich Dispersionen auf Basis dieses Monomers auch in Form von Primärdispersionen zu Polymerfilmen, speziell für pharmazeutische Darreichungsformen, verarbeiten. Überraschenderweise können dabei auch extrem niederviskose Primärdispersionen zu Polymerfilmen verarbeitet werden.

Ein erster Gegenstand der Erfindung ist daher ein Verfahren zur Herstellung einer wässrigen Polymerdispersion Pd), durch radikalische Emulsionspolymerisation eines Monomergemischs M), enthaltend

    a) N,N-Diethylaminoethylmethacrylat, und

    b) wenigstens eine radikalisch polymerisierbare Verbindung, ausgewählt unter Estern $\alpha,\beta$-ethylenisch ungesättigter Mono- und Dicarbonsäuren mit $C_1$-$C_8$-Alkanolen,

in einem wässrigen Medium bei einem pH-Wert von wenigstens 8.

Ein weiterer Gegenstand der Erfindung ist eine wässrige Polymerdispersion Pd), erhältlich durch ein solches Verfahren.

Ein weiterer Gegenstand der Erfindung ist ein Überzugsmittel, enthaltend eine solche wässrige Polymerdispersion Pd) oder eine daraus durch Trocknung erhältliche Polymerzusammensetzung.

Ein weiterer Gegenstand der Erfindung ist ein pharmazeutisches Mittel oder Lebensmittel, enthaltend

    A) eine Polymerzusammensetzung, erhältlich durch Trocknen und/oder Verfilmen einer solchen Polymerdispersion,
    B) wenigstens einen pharmazeutisch akzeptablen Wirkstoff oder einen Nahrungsergänzungssteff, und

C) gegebenenfalls wenigstens einen pharmazeutisch oder im Lebensmittelbereich akzeptablen Hilfsstoff.

Eine spezielle Ausführungsform ist ein pharmazeutisches Mittel oder Lebensmittel in Form einer oralen Darreichungsform, umfassend einen Überzug auf Basis einer solchen wässrigen Polymerdispersion Pd) oder einer daraus durch Trocknung erhältlichen Polymerzusammensetzung.

Erfindungsgemäß erfolgt die Emulsionspolymerisation in einem wässrigen Medium bei einem pH-Wert von wenigstens 8. Darunter wird verstanden, dass der pH-Wert des Polymerisationsgemischs während der Zugabe der Monomeren nicht unter einen pH-Wert von 8 sinkt. Nach der Zugabe der Monomeren, z. B. während einer etwaigen Nachpolymerisation, kann der pH-Wert des wässrigen Mediums auch geringere Werte als 8 annehmen, jedoch nicht geringer als 7,5. Vorzugsweise beträgt der pH-Wert des wässrigen Mediums während der gesamten Herstellung der Polymerdispersion Pd) und auch der pH-Wert der resultierenden Polymerdispersion Pd) wenigstens 8. Vorzugsweise liegt der pH-Wert des wässrigen Mediums in einem Bereich von 8 bis 10, bevorzugt 8,5 bis 9,5. Es ist vorteilhaft, den pH-Wert während der gesamten Polymerisation in den zuvor genannten Bereichen zu halten. Die Einstellung des pH-Werts kann durch Zugabe von pH-regelnden Substanzen, wie Basen, Säuren oder Puffern zum Polymerisationsgemisch erfolgen. Für bestimmte Einsatzbereiche kann die Zugabe von pH-regelnden Substanzen jedoch nicht erwünscht sein. So kann der Einsatz von Salzen oder salzbildenden Komponenten beispielsweise dazu führen, dass die aus den Dispersionen resultierenden Filme eine unerwünscht hohe Durchlässigkeit für darin eingeschlossene Wirkstoffe aufweisen. Dies gilt auch für weitere Salze oder salzbildende Komponenten, wie anionische Emulgatoren, langkettige Fettsäuren, etc. In vielen Fällen ist die Zugabe von pH-regelnden Substanzen auch nicht erforderlich, da das erfindungsgemäß eingesetzte Monomer a), gegebenenfalls in Kombination mit weiteren kationogenen/kationischen Monomeren c) sowie gegebenenfalls Emulgatoren mit ionogenen/ionischen Gruppen dazu führt, dass der pH-Wert in dem gewünschten Bereich liegt.

Zur Einstellung des pH-Werts bei der Polymerisation oder im Anschluss daran eignen sich prinzipiell alle anorganischen oder organischen Basen und Säuren, insbesondere solche, die wasserlöslich sind. Geeignete Basen sind z. B. Alkali- und Erdalkalihydroxide, Ammoniak sowie primäre, sekundäre und tertiäre Amine, wie Triethylamin, sowie Aminoalkohole, wie Triethanolamin, Methyldiethanolamin, Dimethylethanolamin oder 2-Amino-2-methylpropanol. Geeignete Säuren sind z. B. Carbonsäuren, wie Milchsäure, Essigsäure, Citronensäure oder Weinsäure oder anorganische Säuren, wie Phosphorsäure, Diphosphorsäure, Schwefelsäure oder Salzsäure. Der pH-Wert des Polymerisationsgemischs kann vor und während der Polymerisation durch geeignete Messgeräte, beispielsweise durch eine Einstabmesskette, bestimmt werden.

Vorzugsweise wird als Polymerisationsmedium ausschließlich Wasser verwendet.

Im Rahmen der vorliegenden Erfindung umfasst der Ausdruck Alkyl geradkettige und verzweigte Alkylgruppen. Geeignete kurzkettige Alkylgruppen sind z. B. geradkettige oder verzweigte $C_1$-$C_8$-Alkyl-, bevorzugt $C_1$-$C_6$-Alkyl- und besonders bevorzugt $C_1$-$C_4$-Alkylgruppen. Dazu zählen insbesondere Methyl, Ethyl, Propyl, Isopropyl, n-Butyl, 2-Butyl, sec.-Butyl, tert.-Butyl, n-Pentyl, 2-Pentyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 2-Hexyl, 2-Methylpentyl, 3-Methylpentyl, 4-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 2-Ethylbutyl, 1-Ethyl-2-methylpropyl, n-Heptyl, 2-Heptyl, 3-Heptyl, 2-Ethylpentyl, 1-Propylbutyl, n-Octyl, 2-Octyl, 3-Octyl, 2-Ethylhexyl, 1-Propylpentyl, etc.

Geeignete längerkettige $C_9$-$C_{30}$-Alkyl- bzw. $C_9$-$C_{30}$-Alkenylgruppen sind geradkettige und verzweigte Alkyl- bzw. Alkenylgruppen. Bevorzugt handelt es sich dabei um überwiegend lineare Alkylreste, wie sie auch in natürlichen oder synthetischen Fettsäuren und Fettalkoholen sowie Oxoalkoholen vorkommen, die gegebenenfalls zusätzlich einfach, zweifach oder mehrfach ungesättigt sein können. Dazu zählen z. B. n-Nonyl, n-Nonenyl, n-Decyl, n-Decenyl, n-Undecyl, n-Undecenyl, n-Dodecyl, n-Dodecenyl, n-Tridecyl, n-Tridecenyl, n-Tetradecyl, n-Tetradecenyl, n-Pentadecyl, n-Pentadecenyl, n-Hexadecyl, n-Hexadecenyl, n-Heptadecyl, n-Heptadecenyl, n-Octadecyl, n-Octadecenyl, n-Nonadecyl, n-Nonadecenyl etc.

Cycloalkyl steht vorzugsweise für $C_5$-$C_8$-Cycloalkyl, wie Cyclopentyl, Cyclohexyl, Cycloheptyl oder Cyclooctyl.

Aryl umfasst unsubstituierte und substituierte Arylgruppen und steht vorzugsweise für Phenyl, Tolyl, Xylyl, Mesityl, Naphthyl, Fluorenyl, Anthracenyl, Phenanthrenyl, Naphthacenyl und insbesondere für Phenyl, Tolyl, Xylyl oder Mesityl.

Im Folgenden werden Verbindungen, die sich von Acrylsäure und Methacrylsäure ableiten können, teilweise verkürzt durch Einfügen der Silbe "(meth)" in die von der Acrylsäure abgeleitete Verbindung bezeichnet.

Monomer a)

Als Monomer a) wird erfindungsgemäß N,N-Diethylaminoethylmethacrylat eingesetzt.

Zur Herstellung der erfindungsgemäßen wässrigen Polymerdispersionen Pd) wird die Komponente a) vorzugsweise in einer Menge von 25 bis 65 Gew.-%, besonders bevorzugt 30 bis 60 Gew.-%, insbesondere 38 bis 48 Gew.-%,

speziell 43 bis 47 Gew.-%, bezogen auf das Gesamtgewicht der zur Polymerisation eingesetzten Monomere, eingesetzt.

**Monomer b)**

Die Komponente b) ist ausgewählt unter Estern $\alpha,\beta$-ethylenisch ungesättigter Mono- und Dicarbonsäuren mit $C_1$-$C_8$-Alkanolen.

Geeignete Verbindungen b) sind Methyl(meth)acrylat, Methylethacrylat, Ethyl(meth)acrylat, Ethylethacrylat, n-Propyl(meth)acrylat, Isopropyl(meth)acrylat, n-Butyl(meth)acrylat, sec.-Butyl(meth)acrylat, tert.-Butyl(meth)acrylat, tert.-Butylethacrylat, n-Hexyl(meth)acrylat, n-Heptyl(meth)acrylat, n-Octyl(meth)acrylat, 1,1,3,3-Tetramethylbutyl(meth)acrylat und Ethylhexyl(meth)acrylat.

Besonders bevorzugt wird als Komponente b) Methylmethacrylat oder ein Methylmethacrylat enthaltendes Monomergemisch eingesetzt.

Speziell bevorzugt ist es als Komponente b) Methylmethacrylat einzusetzen.

Zur Herstellung der erfindungsgemäßen wässrigen Polymerdispersionen Pd) wird die Komponente b) vorzugsweise in einer Menge von 35 bis 75 Gew.- %, besonders bevorzugt 40 bis 70 Gew.-%, insbesondere 52 bis 62 Gew.-%, speziell 53 bis 57 Gew.-%, bezogen auf das Gesamtgewicht der zur Polymerisation eingesetzten Monomere, eingesetzt.

**Monomer c)**

Die zur Herstellung der Polymerdispersionen Pd) eingesetzten Monomergemische M) können zusätzlich wenigstens ein weiteres Monomer c) enthalten. Die zusätzlichen Monomere c) sind vorzugsweise ausgewählt unter Estern $\alpha,\beta$-ethylenisch ungesättigter Mono- und Dicarbonsäuren mit $C_9$-$C_{30}$-Alkanolen und $C_2$-$C_{30}$-Alkandiolen, Amiden $\alpha,\beta$-ethylenisch ungesättigter Mono- und Dicarbonsäuren mit $C_2$-$C_{30}$-Aminoalkoholen, die eine primäre oder sekundäre Aminogruppe aufweisen, primären Amiden $\alpha,\beta$-ethylenisch ungesättigter Monocarbonsäuren und deren N-Alkyl- und N,N-Dialkylderivaten, N-Vinyllactamen, offenkettigen N-Vinylamidverbindungen, Estern von Vinylalkohol und Allylalkohol mit $C_1$-$C_{30}$-Monocarbonsäuren, Vinylethern, Vinylaromaten, Vinylhalogeniden, Vinylidenhalogeniden, $C_2$-$C_8$-Monoolefinen, ungesättigte Nitrile, nicht aromatischen Kohlenwasserstoffen mit mindestens zwei konjugierten Doppelbindungen und Mischungen davon.

Geeignete zusätzliche Monomere c) sind Ester $\alpha,\beta$-ethylenisch ungesättigter Mono- und Dicarbonsäuren mit $C_9$-$C_{30}$-Alkanolen, wie n-Nonyl(meth)acrylat, n-Decyl(meth)acrylat, n-Undecyl(meth)acrylat, Tridecyl(meth)acrylat, Myristyl(meth)acrylat, Pentadecyl(meth)acrylat, Palmityl(meth)acrylat, Heptadecyl(meth)acrylat, Nonadecyl(meth)acrylat, Arachinyl(meth)acrylat, Behenyl(meth)acrylat, Lignoceryl(meth)acrylat, Cerotinyl(meth)acrylat, Melissinyl(meth)acrylat, Palmitoleinyl(meth)acrylat, Oleyl(meth)acrylat, Linolyl(meth)acrylat, Linolenyl(meth)acrylat, Stearyl(meth)acrylat, Lauryl(meth)acrylat und Mischungen davon.

Geeignete zusätzliche Monomere c) sind weiterhin Ester $\alpha,\beta$-ethylenisch ungesättigter Mono- und Dicarbonsäuren mit $C_2$-$C_{30}$-Alkandiolen, wie 2-Hydroxyethylacrylat, 2-Hydroxyethylmethacrylat, 2-Hydroxyethylethacrylat, 2-Hydroxypropylacrylat, 2-Hydroxypropylmethacrylat, 3-Hydroxypropylacrylat, 3-Hydroxypropylmethacrylat, 3-Hydroxybutylacrylat, 3-Hydroxybutylmethacrylat, 4-Hydroxybutylacrylat, 4-Hydroxybutylmethacrylat, 6-Hydroxyhexylacrylat, 6-Hydroxyhexylmethacrylat, 3-Hydroxy-2-ethylhexylacrylat, 3-Hydroxy-2-ethylhexylmethacrylat etc.

Geeignete zusätzliche Monomere c) sind weiterhin primäre Amide $\alpha,\beta$-ethylenisch ungesättigter Monocarbonsäuren und deren N-Alkyl- und N,N-Dialkylderivate, wie Acrylsäureamid, Methacrylsäureamid, N-Methyl(meth)acrylamid, N-Ethyl(meth)acrylamid, N-Propyl(meth)acrylamid, N-(n-Butyl)(meth)acrylamid, N-(tert.-Butyl)(meth)acrylamid, N-(n-Octyl)(meth)acrylamid, N-(1,1,3,3-Tetramethylbutyl)(meth)acrylamid, N-Ethylhexyl(meth)acrylamid, N-(n-Nonyl)(meth)acrylamid, N-(n-Decyl)(meth)äcrylamid, N-(n-Undecyl)(meth)acrylamid, N-Tridecyl(meth)acrylamid, N-Myristyl(meth)acrylamid, N-Pentadecyl(meth)acrylamid, N-Palmityl(meth)acrylamid, N-Heptadecyl(meth)acrylamid, N-Nonadecyl(meth)acrylamid, N-Arachinyl(meth)acrylamid, N-Behenyl(meth)acrylamid, N-Lignoceryl(meth)acrylamid, N-Cerotinyl(meth)acrylamid, N-Melissinyl(meth)acrylamid, N-Palmitoleinyl(meth)acrylamid, N-Oleyl(meth)acrylamid, N-Linolyl(meth)acrylamid, N-Linolenyl(meth)acrylamid, N-Stearyl(meth)acrylamid, N-Lauryl(meth)acrylamid, N,N-Dimethyl(meth)acrylamid, N,N-Diethyl(meth)acrylamid, Morpholinyl(meth)acrylamid.

Als zusätzliche Monomere c) eignen sich weiterhin N-Vinyllactame und deren Derivate, die z. B. einen oder mehrere $C_1$-$C_6$-Alkylsubstituenten, wie Methyl, Ethyl, n-Propyl, Isopropyl, n-Butyl, sec.-Butyl, tert.-Butyl etc., aufweisen können. Dazu zählen z. B. N-Vinylpyrrolidon, N-Vinylpiperidon, N-Vinylcaprolactam, N-Vinyl-5-methyl-2-pyrrolidon, N-Vinyl-5-ethyl-2-pyrrolidon, N-Vinyl-6-methyl-2-piperidon, N-Vinyl-6-ethyl-2-piperidon, N-Vinyl-7-methyl-2-caprolactam, N-Vinyl-7-ethyl-2-caprolactam etc. Bevorzugt werden N-Vinylpyrrolidon und N-Vinylcaprolactam eingesetzt.

Als Monomere c) geeignete offenkettige N-Vinylamidverbindungen sind beispielsweise N-Vinylformamid, N-Vinyl-N-methylformamid, N-Vinylacetamid, N-Vinyl-N-methylacetamid, N-Vinyl-N-ethylacetamid, N-Vinylpropionamid, N-Vinyl-N-methylpropionamid und N-Vinylbutyramid.

Geeignete zusätzliche Monomere c) sind weiterhin Vinylacetat, Vinylpropionat, Vinylbutyrat und Mischungen davon.

Geeignete zusätzliche Monomere c) sind weiterhin Ethylen, Propylen, Isobutylen, Butadien, Styrol, $\alpha$-Methylstyrol, Acrylnitril, Methacrylnitril, Vinylchlorid, Vinylidenchlorid, Vinylfluorid, Vinylidenfluorid und Mischungen davon.

Die zuvor genannten zusätzlichen Monomere c) können einzeln oder in Form von beliebigen Mischungen eingesetzt werden.

Zur Herstellung der erfindungsgemäßen wässrigen Polymerdispersionen Pd) wird die Komponente c) vorzugsweise in einer Menge von 0 bis 80 Gew.-%, bezogen auf das Gesamtgewicht der zur Polymerisation eingesetzten Monomere, eingesetzt. Eine spezielle Ausführungsform betrifft Polymerdispersionen Pd), die kein zusätzliches Monomer c) einpolymerisiert enthalten. Soweit vorhanden, wird die Komponente c) vorzugsweise in einer Menge von 0,1 bis 70 Gew.-%, besonders bevorzugt 1 bis 60 Gew.-%, insbesondere 5 bis 50 Gew.-%, bezogen auf das Gesamtgewicht der zur Polymerisation eingesetzten Monomere, eingesetzt.

Vorzugsweise wird kein Monomer c) verwendet.

Monomer d)

Die zur Herstellung der Polymerdispersionen Pd) eingesetzten Monomergemische M) können zusätzlich zur Verbindung a) wenigstens eine weitere, davon verschiedene Verbindung d) mit einer radikalisch polymerisierbaren $\alpha,\beta$-ethylenisch ungesättigten Doppelbindung und mindestens einer kationogenen und/oder kationischen Gruppe pro Molekül einpolymerisiert enthalten.

Bevorzugt handelt es sich bei den kationogenen bzw. kationischen Gruppen der Komponente d) um stickstoffhaltige Gruppen, wie primäre, sekundäre und tertiäre Aminogruppen sowie quaternäre Ammoniumgruppen. Vorzugsweise handelt es sich bei den stickstoffhaltigen Gruppen um tertiäre Aminogruppen oder quaternäre Ammoniumgruppen. Geladene kationische Gruppen lassen sich aus den Aminstickstoffen entweder durch Protonierung, z. B. mit einwertigen oder mehrwertigen Carbonsäuren, wie Milchsäure oder Weinsäure, oder Mineralsäuren, wie Phosphorsäure, Schwefelsäure und Salzsäure, oder durch Quaternisierung, z. B. mit Alkylierungsmitteln, wie $C_1$-$C_4$-Alkylhalogeniden oder -sulfaten, erzeugen. Beispiele solcher Alkylierungsmittel sind Ethylchlorid, Ethylbromid, Methylchlorid, Methylbromid, Dimethylsulfat und Diethylsulfat.

Geeignete Verbindungen d) sind z. B. die von DEAEMA verschiedene Ester von $\alpha,\beta$-ethylenisch ungesättigten Mono- und Dicarbonsäuren mit Aminoalkoholen. Bevorzugte Aminoalkohole sind $C_2$-$C_{12}$-Aminoalkohole, welche am Aminstickstoff $C_1$-$C_8$-mono- oder dialkyliert sind. Als Säurekomponente dieser Ester eignen sich z. B. Acrylsäure, Methacrylsäure, Fumarsäure, Maleinsäure, Itaconsäure, Crotonsäure, Maleinsäureanhydrid, Monobutylmaleat und Gemische davon. Bevorzugt werden als Säurekomponente dieser Ester Acrylsäure, Methacrylsäure und deren Gemische eingesetzt.

Geeignete zusätzliche Verbindungen d) sind N,N-Dimethylaminomethyl(meth)acrylat, N,N-Dimethylaminoethyl(meth)acrylat, N,N-Diethylaminoethylacrylat, N,N-Dimethylaminopropyl(meth)acrylat, N,N-Diethylaminopropyl(meth)acrylat und N,N-Dimethylaminocyclohexyl(meth)acrylat.

Geeignete Monomere d) sind weiterhin die Amide der zuvor genannten $\alpha,\beta$-ethylenisch ungesättigten Mono- und Dicarbonsäuren mit Diaminen, welche mindestens eine primäre oder sekundäre Aminogruppe aufweisen. Bevorzugt sind Diamine, die eine tertiäre und eine primäre oder sekundäre Aminogruppe aufweisen.

Dazu zählen N-[2-(Dimethylamino)ethyl]acrylamid, N-[2-(Dimethylamino)ethyl]methacrylamid, N-[3-(Dimethylamino)propyl]acrylamid, N-[3-(Dimethylamino)propyl]methacrylamid, N-[4-(Dimethylamino)butyl]acrylamid, N-[4-(Dimethylamino)-butyl]methacrylamid, N-[2-(Diethylamino)ethyl]acrylamid, N-[4-(Dimethylamino)cyclohexyl]acrylamid, N-[4-(Dimethylamino)cyclohexyl]methacrylamid etc.

Geeignete Monomere d) sind weiterhin N,N-Diallylamine und N,N-Diallyl-N-alkylamine und deren Säureadditionssalze und Quaternisierungsprodukte. Alkyl steht dabei vorzugsweise für $C_1$-$C_{24}$-Alkyl. Bevorzugt sind N,N-Diallyl-N-methylamin und N,N-Diallyl-N,N-dimethylammonium-Verbindungen, wie z. B. die Chloride und Bromide.

Geeignete Monomere d) sind weiterhin vinyl- und allylsubstituierte Stickstoffheterocyclen, wie N-Vinylimidazol, N-Vinyl-2-methylimidazol, vinyl- und allylsubstituierte heteroaromatische Verbindungen, wie 2- und 4-Vinylpyridin, 2- und 4-Allylpyridin, und die Salze davon.

Zur Herstellung der erfindungsgemäßen wässrigen Polymerdispersionan Pd) wird die Komponente d), soweit vorhanden, vorzugsweise in einer Menge eingesetzt, dass die Summe der Mengen der Komponente a) und der Komponente d) In einem Bereich von 25 bis 65 Gew.- %, besonders bevorzugt 30 bis 60 Gew.-%, bezogen auf das Gesamtgewicht der zur Polymerisation eingesetzten Monomere, liegt.

Zur Herstellung der erfindungsgemäßen wässrigen Polymerdispersionen Pd) wird die Komponente d) vorzugsweise in einer Menge von 0 bis 50 Gew.-%, bezogen auf das Gesamtgewicht der zur Polymerisation eingesetzten Monomere, eingesetzt.

Wie bereits ausgeführt, wurde überraschenderweise gefunden, dass die erfindungsgemäßen und erfindungsgemäß eingesetzten Polymerdispersionen Pd) auf Basis von DEAEMA (Komponente a)) über ein besonders gutes Eigenschaftsprofil verfügen. Dieses Eigerischaftsprofil kann In der Regel ohne den Einsatz weiterer Monomere mit kationogenen/kationischen Gruppen erzielt werden. Eine spezielle Ausführungsform betrifft daher Polymerdispersionen Pd), die kein zusätzliches Monomer d) einpolymerisiert enthalten.

Soweit vorhanden, wird die Komponente d) vorzugsweise in einer Menge von 0,1 bis 40 Gew.-%, besonders bevorzugt 1 bis 30 Gew.-%, insbesondere 2 bis 25 Gew.-%, bezogen auf das Gesamtgewicht der zur Polymerisation eingesetzten Monomere, eingesetzt.

Besonders bevorzugt ist ein Verfahren wie zuvor beschrieben, bei dem ein Monomerengemisch M) eingesetzt wird, das

● 25 bis 65 Gew.-%, bezogen auf das Gesamtgewicht der zur Polymerisation eingesetzten Monomere, N,N-Diethylaminoethylmethacrylat a) und

● 35 bis 75 Gew.-%, bezogen auf das Gesamtgewicht der zur Polymerisation eingesetzten Monomere, wenigstens einer Verbindung b)

enhält.

Ebenso besonders bevorzugt ist ein Verfahren wie zuvor beschrieben, bei dem ein Monomerengemisch M) eingesetzt wird, das aus

● 43 bis 47 Gew.-%, bezogen auf das Gesamtgewicht der zur Polymerisation eingesetzten Monomere, N,N-Diethylaminoethylmethacrylat a) und

● 53 bis 57 Gew.-%, bezogen auf das Gesamtgewicht der zur Polymerisation eingesetzten Monomere, wenigstens einer Verbindung b)

besteht.

In einer besonders bevorzugten Ausführungsform des erfindungsgemäßen Verfahrens wird ein Monomergemisch M) eingesetzt wird, das aus

43 bis 47 Gew.- %, bezogen auf das Gesamtgewicht der zur Polymerisation eingesetzten Monomere, N,N-Diethylaminoethylmethacrylat a), und

- 53 bis 57 Gew.- %, bezogen auf das Gesamtgewicht der zur Polymerisation eingesetzten Monomere, wenigstens einer Verbindung b), insbesondere Methylmethacrylate,

besteht.

Regler

Die radikalische Polymerisation des Monomergemischs M) kann in Gegenwart mindestens eines Reglers erfolgen. Regler werden vorzugsweise in einer Einsatzmenge von 0,0005 bis 5 Gew.-%, besonders bevorzugt von 0,001 bis 2,5 Gew.-% und insbesondere von 0,01 bis 1,5 Gew.-%, bezogen auf das Gesamtgewicht der zur Polymerisation eingesetzten Monomere, eingesetzt.

Als Regler (Polymerisationsregler) werden allgemein Verbindungen mit hohen Übertragungskonstanten bezeichnet. Regler beschleunigen Kettenübertragungsreaktionen und bewirken damit eine Herabsetzung des Polymerisationsgrades der resultierenden Polymeren, ohne die Bruttoreaktionsgeschwindigkeit zu beeinflussen. Bei den Reglern kann man zwischen mono-, bi- oder polyfunktionalen Regler unterscheiden je nach Anzahl der funktionellen Gruppen im Molekül, die zu einen oder mehreren Kettenübertragungsreaktionen führen können. Geeignete Regler werden beispielsweise ausführlich beschrieben von K. C. Berger und G. Brandrup in J. Brandrup, E. H. Immergut, Polymer Handbook, 3. Aufl., John Wiley & Sons, New York, 1989, S. II/81 - II/141.

Bevorzugt werden als Regler Verbindungen eingesetzt, die Schwefel in gebundener Form enthalten.

Geeignet als Polymerisationsregler sind weiterhin Thiole (Verbindungen, die Schwefel in Form von SH-Gruppen erhalten, auch als Mercaptane bezeichnet). Bevorzugt sind als Regler mono-, bi- und polyfunktionale Mercaptane, Mercaptoalkohole und/oder Mercaptocarbonsäuren. Beispiele für diese Verbindungen sind Alkylthioglykolate, Ethylhexylthioglykolat, Cystein, 2-Mercaptoethanol, 3-Mercapto-1-propanol, 3-Mercaptopropan-1,2-diol, 4-Mercapto-1-butanol, Mercaptoessigsäure, 3-Mercaptopropionsäure, Mercaptobernsteinsäure, Thioglycerin, Thioessigsäure, Thioharnstoff und Alkylmercaptane wie n-Butylmercaptan, n-Hexylmercaptan, n-Dodecylmercaptan oder tert.-Dodecylmercaptan.

Alle genannten Regler können einzeln oder in Kombination miteinander eingesetzt werden.

Zur Herstellung der Polymerisate können die Monomeren mit Hilfe von Radikale bildenden Initiatoren polymerisiert werden.

Als Initiatoren für die radikalische Polymerisation können die hierfür üblichen Peroxo- und/oder Azo-Verbindungen eingesetzt werden, beispielsweise Alkali- oder Ammoniumperoxidisulfate, Diacetylperoxid, Dibenzoylperoxid, Succinylperoxid, Di-tert.-butylperoxid, tert.-Butylperbenzoat, tert.-Butylperpivalat, tert.-Butylperoxy-2-ethylhexanoat, tert.-Butytpermaleinat, Cumolhydroperoxid, Düsopropylperoxydicarbamat, Bis-(o-toluoyl)peroxid, Didecanoylperoxid, Dioctanoylperoxid, Dilauroylperoxid, tert.-Butylperisobutyrat, tert.-Butylperacetat, Di-tert.-Amylperoxid, tert.-Butylhydroperoxid, Azo-bis-isobutyronitril, Azo-bis-(2-amidinopropan)dihydrochlorid oder 2,2'-Azo-bis-(2-methyl-butyronitril). Geeignet sind auch Initiatormischungen oder Redox-Initiator-Systeme, wie z. B. Ascorbinsäure/Eisen(II)sulfat/Natriumperoxodisulfat, tert.-Butylhydroperoxid/Natriumdisulfit, tert.-Butylhydroperoxid/Natriumhydroxymethansulfinat, $H_2O_2/Cu^1$.

Zur Herstellung der erfindungsgemäßen und erfindungsgemäß verwendeten wässrigen Polymerdispersion Pd), wird vorzugsweise wenigstens ein anorganisches Peroxid als Initiator eingesetzt. Besonders bevorzugt ist der Initiator ausgewählt ist unter Ammonium- und Alkaliperoxodisulfaten. Ganz besonders bevorzugt sind Natriumperoxodisulfat oder Kaliumperoxodisulfat, Der Einsatz dieser Peroxodisulfate erfolgt vorzugsweise in Form einer wässrigen Lösung, die eine Peroxodisulfatkonzentration in einem Bereich von 0,5 bis 20 Gew.-%, besonders bevorzugt von 2 bis 10 Gew.-%, aufweist.

Besonders bevorzugt ist ein Verfahren wie zuvor beschrieben, wobei zur radikalischen Emulsionspolymerisation wenigstens ein anorganisches Peroxid als Initiator eingesetzt wird, wobei der Initiator ausgewählt ist unter Ammonium- und Alkaliperoxodisulfaten.

Die Polymerisation erfolgt, wie bereits ausgeführt, bei einem alkalischen pH-Wert. Dabei ist es besonders vorteilhaft, nicht nur das Polymerisationsmedium möglichst während der gesamten Polymerisation in dem erwünschten pH-Bereich zu halten, sondern auch den pH-Wert des Initiatorzulaufs auf einen nicht zu sauren pH-Wert einzustellen. Wird zur Polymerisation eine wässrige Lösung wenigstens eines anorganischen Peroxids als Initiator eingesetzt, so weist diese einen pH-Wert von vorzugsweise größer als 2, besonders bevorzugt von größer als 3, insbesondere von größer als 4, auf. Bevorzugte Peroxodisuifatkonzentrationen sind die zuvor genannten. Zur Einstellung des pH-Werts eignen sich prinzipiell alle anorganischen oder organischen Basen, insbesondere solche, die außer einer etwaigen Salzbildung keine Reaktion mit den Monomeren eingehen. Geeignete Basen sind z. B. Alkali- und Erdalkalihydroxide, tertiäre Amine, wie Triethylamin, sowie Aminoalkohole, wie Triethanolamin, Methyldiethanolamin oder Dimethylethanolamin. Bevorzugt wird zur Einstellung des pH-Werts NaOH oder KOH eingesetzt. Alternativ, wird zur radikalischen Emulsionspolymerisation eine wässrige Natriumperoxodisulfat-Lösung oder eine wässrige Kaliumperoxodisulfat-Lösung als Initiator eingesetzt wird, die unmittelbar vor ihrem Einsatz zur Polymerisation hergestellt wird und deren pH somit in einem vorteilhaften Bereich liegt, Unmittelbar vor dem Einsatz zur Polymerisation hergestellt bedeutet, dass die Herstellung nicht länger als 24 Stunden, bevorzugt nicht länger als 12 Stunden, insbesondere nicht länger als 6 Stunden vor dem Einsatz zur Polymerisation erfolgt.

Besonders bevorzugt ist ein Verfahren wie zuvor beschrieben, wobei zur radikalischen Emulsionspolymerisation eine wässrige Natriumperoxodisulfat-Lösung oder Kaliumperoxodisulfat-Lösung als Initiator eingesetzt wird, die unmittelbar vor ihrem Einsatz zur Polymerisation hergestellt wird oder deren pH-Wert vor ihrem Einsatz zur Polymerisation durch Zugabe wenigstens einer Base auf einen Wert von größer als 2, bevorzugt von größer als 3, insbesondere von größer als 4, eingestellt wird.

Die Herstellung der erfindungsgemäßen und erfindungsgemäß verwendeten wässrigen Polymerdispersion Pd), erfolgt üblicherweise in Gegenwart wenigstens einer grenzflächenaktiven Verbindung.

Geeignete grenzflächenaktive Verbindungen sind die üblicherweise bei der Emulsionspolymerisation als Dispergiermittel eingesetzten Schutzkolloide und Emulgatoren, wie sie z. B. in Houben-Weyl, Methoden der organischen Chemie, Band X!V/1, Makromolekulare Stoffe, Georg-Thieme-Verlag, Stuttgart, 1961, S. 411 bis 420 beschrieben sind. Als Emulgatoren sind sowohl anionische als auch nichtionische Emulgatoren geeignet. Vorzugsweise werden als grenzflächenaktive Substanzen Emulgatoren eingesetzt, deren relative Molekulargewichte üblicherweise unterhalb 2500 g/mol liegen.

Brauchbare nichtionische Emulgatoren sind araliphatische oder aliphatische nichtionische Emulgatoren, beispielsweise ethoxylierte Mono-, Di- und Trialkylphenole (EO-Grad: 3 bis 50, Alkylrest: $C_4$-$C_{10}$), Ethoxylate langkettiger Alkohole (EO-Grad: 3 bis 100, Alkylrest: $C_8$-$C_{38}$) sowie Polyethylenoxid/Polypropylenoxid-Homo- und Copolymere. Diese können die Alkylenoxideinheiten statistisch verteilt oder in Form von Blöcken einpolymerisiert enthalten. Gut geeignet sind z. B. EO/PO-Blockcopolymere. Bevorzugt werden Ethoxylate langkettiger Alkanole (Alkylrest $C_1$-$C_{30}$, mittlerer Ethoxyüerungsgrad 5 bis 100) und darunter besonders bevorzugt solche mit einem linearen $C_{12}$-$C_{20}$-Alkylrest und einem mittleren Ethoxylierungsgrad von 10 bis 50 sowie ethoxylierte Monoalkylphenole, eingesetzt.

Besonders bevorzugte nichtionische Emulgatoren sind Fettalkoholalkoxilate von Alkoholen mit 12 bis 30 Kohlenstoffatomen, Diese weisen vorzugsweise 10 bis 30 Ethylenoxideinheiten auf. In einer speziellen Ausführung ist der nichtionische Emulgator dann ausgewählt ist unter Ethoxilaten von Cetylalkohol und/oder Stearylalkohol mit jeweils 10 bis 30 Ethylenoxideinheiten (wie z. B. 20 Ethylenoxideinheiten).

Geeignete anionische Emulgatoren sind beispielsweise Alkali- und Ammoniumsalze von Alkylsulfaten (Alkylrest: $C_8$-$C_{22}$), von Schwefelsäurehalbestern ethoxylierter Alkanole (EO-Grad: 2 bis 50, Alkylrest: $C_{12}$-$C_{18}$) und ethoxylierter Alkylphenole (EO-Grad: 3 bis 50, Alkylrest: $C_4$-$C_9$), von Alkylsulfonsäuren (Alkylrest: $C_{12}$-$C_{18}$) und von Alkylarylsulfonsäuren (Alkylrest: $C_9$-$C_{18}$). Weitere geeignete Emulgatoren finden sich in Houben-Weyl, Methoden der organischen Chemie, Band XIV/1, Makromolekulare Stoffe, Georg-Thieme-Verlag, Stuttgart, 1961, S. 192-208). Als anionische Emulgatoren sind ebenfalls Bis(phenylsulfonsäure)ether bzw. deren Alkali- oder Ammoniumsalze, die an einem oder beiden aromatischen Ringen eine $C_4$-$C_{24}$-Alkylgruppe tragen, geeignet. Diese Verbindungen sind allgemein bekannt, z. B. aus der US-A-4,269,749, und im Handel erhältlich, beispielsweise als Dowfax® 2A1 (Dow Chemical Company).

Besonders geeignete anionische Emulgatoren sind Natriumlaurylsulfat, Natrium(cetyllstearyl)sulfat, etc.

Bevorzugt erfolgt die radikalische Emulsionspolymerisation in Gegenwart wenigstens eines nichtionischen Emulgators oder eines Emulgatorgemischs, das wenigstens einen anionischen Emulgator und wenigstens einen nichtionischen Emulgator umfasst. Dazu zählen z. B. Gemische aus Natriumlaurylsulfat und den zuvor genannten Fettalkoholalkoxilaten von Alkoholen mit 12 bis 30 Kohlenstoffatomen und mit jeweils 10 bis 30 Ethylenoxideinheiten.

In einer bevorzugten Ausführungsform erfolgt die radikalische Emulsionspolymerisation in Gegenwart wenigstens eines nichtionischen Emulgators oder eines Emulgatorgemisches, das wenigstens einen anionischen Emulgator und wenigstens einen nichtionischen Emulgator enthält. Vorzugsweise umfasst der nichtionische Emulgator wenigstens ein Fettalkoholalkoxilat von Alkoholen mit 12 bis 30 Kohlenstoffatomen. Insbesondere ist der nichtionische Emulgator ausgewählt unter Ethoxilaten von Cetylalkohol und/oder Stearylalkohol mit jeweils 10 bis 30 Ethylenoxideinheiten.

Die Gesamtmenge an Emulgator beträgt im Allgemeinen etwa 0,05 bis 6 Gew.-%, bevorzugt 1 bis 5 Gew.-%, bezogen auf die Menge an zu polymerisierenden Monomeren. Das Gewichtsverhältnis von anionischen Emulgatoren zu nichtionischen Emulgatoren liegt vorzugsweise in einem Bereich von 1: 2 bis 2 : 1 und beträgt besonders bevorzugt etwa 1 : 1 (wie z. B. 1,3 : 1 bis 1 : 1,3). In einer speziellen Ausführungsform beträgt der Gehalt an nichtionischen Emulgatoren höchstens 4 Gew.-%, bezogen auf die Menge an zu polymerisierenden Monomeren. Der Emulgatorgehalt der Polymerdispersionen Pd) kann nach der Polymerisation noch variiert werden. So kann sich z. B. der Emulgatorgehalt verringern, wenn die Dispersion einer Ultrafiltration unterzogen wird. Dabei können dann Dispersionen mit einem Gesamtemulgatorgehalt von weniger als 5 Gew.-%, speziell weniger als 4 Gew.-%, noch spezieller weniger als 3 Gew.-%, bezogen auf die Monomermenge resultieren.

Zur Herstellung der Polymerdispersionen Pd) können weiterhin von den zuvor genannten Emulgatoren verschiedene Dispergiermitteln D) eingesetzt werden. Dazu zählen z. B. polymere Dispergiermittel, die, soweit vorhanden, im Allgemeinen in Mengen von 0 bis 10 Gew.-%, bevorzugt 0,01 bis 5 Gew.-%, bezogen auf die Menge an zu polymerisierenden Monomeren eingesetzt werden. Bevorzugt werden keine von den zuvor genannten Emulgatoren verschiedene Dispergiermittel eingesetzt.

Den Polymerdispersionen Pd) können weiterhin übliche Hilfs- und Zusatzstoffe zugesetzt werden.

Dazu zählen beispielsweise den pH-Wert einstellende Substanzen, Reduktions- und Bleichmittel, wie z. B. die Alkalimetallsalze der Hydroxymethansulfinsäure (z. B. Rongallit® C der BASF Aktiengesellschaft), Komplexbildner, Desodorantien, Geschmacksstoffe, Geruchsstoffe, Desinfektionsmittel, Konservierungsmittel und Viskositätsmodifizierer, wie Alkohole, z. B. Glycerin, Methanol, Ethanol, tert.-Butanol, Glykol, Propylenglykol, oder 2-Pyrrolidon, N-Methyl -2-pyrrolidon, Polyethylenglykol 400 etc.

Als Konservierungsmittel sind prinzipiell alle Stoffe geeignet, die in einem pH-Bereich größer 7 wirksam sind. Solche Stoffe sind beispielsweise in "Praxis der Sterilisation, Desinfektion - Konservierung", Karl Heinz Wallhäußer 5. überarbeitete Auflage, Georg Thieme Verlag Stuttgart, 1995, S. 465 - 652 aufgelistet. Besonders geeignete Stoffe sind: Benzylalkohol, 2,4-Dichlorbenzylalkohol, Phenylethylalkohol, 2-Phenoxyethanol, 2-Phenoxy-1-propanol, Chlorphenesin, Propylenglykol, Formaldehyd, Acetaldehyd, Acrolein, Glyoxal, Glutaraldehyd, p-Hydroxybenzoesäuremethylester, p-Hydroxybenzoesäureethylester, p-Hydroxybenzoesäurepropylester, p-Hydroxybenzoesäurebutylester, p-Hydroxybenzoesäurebenzylester, Phenol, Chlorphenole, quartäre Ammoniumverbindungen wie z. B. Benzalkoniumchlorid und Cetylpyridiniumchlorid, Quaternium 15, Kresole, Chlorkresole, Silber und Silbersalze, organische Quecksilberverbindungen wie z. B. Phenylquecksilberacetat, Thiomersal, Sulfomerthiolate, Chlor, Chlordioxid, Hypochlorite, Jod, Hypojodite, Wasserstoffperoxid. Benzoylperoxid, 1,3-Dimethylol-5,5-dimethylhydantoin, und Mischungen davon.

Diese Hilfs- und Zusatzstoffe können den Polymerdispersionen in der Vorlage, einem der Zuläufe oder nach Abschluss der Polymerisation zugesetzt werden.

Die Polymerisation erfolgt im Allgemeinen bei Temperaturen in einem Bereich von 0 bis 150 °C, bevorzugt 20 bis 100 °C, besonders bevorzugt 55 bis 95 °C. Die Polymerisation erfolgt vorzugsweise bei Normaldruck, möglich ist jedoch auch eine Polymerisation unter erhöhtem Druck, beispielsweise dem Eigendruck der zur Polymerisation eingesetzten Komponenten. Ein bevorzugter Druckbereich ist 1 bis 5 bar. In einer bevorzugten Ausführung erfolgt die Polymerisation in Gegenwart wenigstens eines Inertgases, wie z. B. Stickstoff oder Argon. Die eingesetzten Inertgase sind dabei vorzugsweise wasserfrei.

Die Polymerisation erfolgt in Form eines Zulaufverfahrens. Die einzelnen Zuläufe können dabei der Polymerisationszone kontinuierlich oder in Stufen- oder Gradientenfahrweise zugeführt werden. In einer möglichen Ausführung erfolgt die Polymerisation als Zulaufverfahren, bei dem man einen Teil des Polymerisationsansatzes (jedoch vorzugsweise keine Monomere) vorlegt und die übrigen Komponenten ganz oder teilweise, absatzweise oder kontinuierlich, gemeinsam oder in getrennten Zuläufen zu der Vorlage gibt.

In einer bevorzugten Ausführung erfolgt die Zugabe der Komponenten von unten in den Polymerisationsreaktor (getaucht).

Bevorzugt wird in dem erfindungsgemäßen Verfahren kein Monomer in der zur Reaktion eingesetzten Polymerisationszone vorgelegt. Des Weiteren erfolgt die radikalische Emulsionspolymerisation vorzugsweise nicht in Gegenwart eines Saatlatex.

Bevorzugt wird ein Teil der eingesetzten Emulgatoren und wenigstens ein Teil des wässrigen Mediums in einer Polymerisationszone vorgelegt, auf die Polymerisationstemperatur erwärmt und anschließend der Rest des Polymerisationsansatzes über einen oder über mehrere räumlich getrennte Zuläufe der Polymerisationszone zugeführt. Die Zuführung von Monomeren und/oder Initiator erfolgt dabei zweckmäßigerweise nach Maßgabe ihres Verbrauchs, d. h. unter Aufrechterhaltung der Polymerisation, Üblicherweise werden dabei Polymerisationsinitiator und Monomere in getrennten Zuläufen zugegeben. Die Zuführung der Monomere kann prinzipiell einzeln oder in Form von Gemischen erfolgen.

Besonders bevorzugt ist ein Verfahren wie zuvor beschrieben, wobei vor Beginn der radikalischen Emulsionspolymerisation kein Monomer in der Polymerisationszone vorgelegt wird.

Ebenfalls besonders bevorzugt ist ein Verfahren wie zuvor beschrieben, wobei die radikalische Emulsionspolymerisation nicht in Gegenwart eines Saatlatex erfolgt.

Vorzugsweise wird das N,N-Diethylaminoethylmethacrylat (Komponente a)) bis zu seinem Einsatz zur Polymerisation unter im Wesentlichen wasserfreien Bedingungen gehandhabt. Dazu erfolgen z. B. die Lagerung und/oder die Zuführung der Komponente a) in die Polymerisationszone im Wesentlichen unter Wasserausschluss. Das Gleiche gilt für Monomergemische, die N,N-Diethylaminoethylmethacrylat enthalten. Geeignete Verfahren zum Abfüllen, Lagern und Fördern von feuchtigkeitsempfindlichen Stoffen sind dem Fachmann bekannt. Dazu zählt z. B. ein Ausschluss von Wasser beim Handhaben der Komponente a), als auch das vorherige Trocknen von Geräteoberfächen, die mit der Komponente a) in Kontakt kommen. Der Ausdruck "unter im Wesentlichen wasserfreien Bedingungen" bedeutet im Rahmen der Erfindung, dass der Kontakt mit geringen Wassermengen, wie er z. B. durch Kontakt mit der Luftfeuchtigkeit beim Umfüllen erfolgt, in der Regel unkritisch ist.

In einer speziellen Ausführung des erfindungsgemäßen Verfahrens wird das N,N-Diethylaminoethylmethacrylat a) unmittelbar vor seinem Einsatz zur Polymerisateon in einer Mischvorrichtung mit Wasser vermischt. Der Ausdruck "unmittelbar vor seinem Einsatz zur Polymerisation" bedeutet dabei, dass das Vermischen mit Wasser höchstens 1 Stunde, vorzugsweise höchstens 10 Minuten, besonders bevorzugt höchstens 1 Minute, vor dem Einsatz zur Polymerisation erfolgt. Vorzugsweise erfolgt das Vermischen dazu auch räumlich unmittelbar vor Eintritt in die Reaktionszone. Vorzugsweise wird das N,N-Diefihylaminoethylmethacrylat a) in der Mischvorrichtung mit Wasser, wenigstens einem Emulgator und gegebenenfalls wenigstens einem weiteren Monomer unter Erhalt einer Monomerenemulsion vermischt.

Besonders bevorzugt ist eine Ausführungsform des erfindungsgemäßen Verfahrens, wobei das N,N-Diethylaminoethylmethacrylat a) unmittelbar vor seinem Einsatz zur Polymerisation in einer Mischvorrichtung mit Wasser, wenigstens einem Emulgator und gegebenenfalls wenigstens einem weiteren Monomer unter Erhalt einer Monomerenemulsion vermischt wird.

Das erfindungsgemäße Verfahren dient der Herstellung von Copolymerisaten, wobei die einzelnen Comonomere jeweils einzeln oder in Form von beliebigen Gemischen in mindestens einer Mischvorrichtung zur Emulsion vermischt werden können. Vorzugsweise werden die zur Polymerisation eingesetzten Comonomere in einer Mischvorrichtung zur Emulsion vermischt. Die Zuführung mehrerer Monomere in die Mischvorrichtung kann separat oder in Gemischen erfolgen, die z. B. durch Zusammenführen der Finzelzuläufe in einer gemeinsamen Rohrleitung erzeugt werden können. Vorzugsweise wird der Mischvorrichtung ein wasserhaltiger Zulauf zugeführt, dem bereits vor Eintritt in die Mischvorrichtung wenigstens eine oberflächenaktive Substanz (Emulgator) zugesetzt wurde.

Die Zugabe des Initiators erfolgt über einen separaten Zulauf, im Allgemeinen in wässriger Phase, wobei jedoch Monomerzulauf und Initiatorzulauf vor Eintritt in die Mischvorrichtung vereinigt werden können. Alternativ kann der Initiator auch unabhängig vom Monomerzulauf direkt in den Reaktor gegeben werden.

Gegebenenfalls kann die Zugabe weiterer Komponenten zur Reaktionszone, die im Folgenden noch genauer definiert werden, je nach Verträglichkeit gemeinsam mit einem der vorgenannten Zuläufe oder separat In reiner Form, als Lösung In Wasser oder auch in einem geeigneten Lösungsmittel erfolgen.

Als Mischvorrichtung können in dem erfindungsgemäßen Verfahren ein oder mehrere Mischer verwendet werden, wobei es sich um Mischer gleicher oder verschiedener Bauart handeln kann, die in beliebiger Reihenfolge, Anordnung und Kombination verwendet werden, wie z. B. Reihenanordnung aller Mischer, Kombination aus Parallel- und Reihenanordnung oder Parallelanordnung aller Mischer. Werden mehrere Mischer verwendet, so ist die Reihenanordnung bevorzugt. Geeignete Mischer sind dynamische Mischer, deren Mischorgane bewegliche Teile enthalten, und statische Mischer, d. h. Mischorgane ohne bewegte Teile im Inneren. Bevorzugt sind Mischer, die insbesondere nach dem Inline-Prinzip arbeiten. Geeignete Mischer werden z. B. in A. Echte, Handbuch der technischen Polymerchemie, VCH Verlagsgesellschaft Weinheim, S. 104 ff. (1993) beschrieben.

In einer bevorzugten Ausführungsform des erfindungsgemäßen Verfahrens wird eine Mischvorrichtung eingesetzt, die wenigstens einen dynamischen Mischer umfasst.

Geeignete dynamische Inline-Mischer sind z. B. die in ZFL-Zeitschrift für Lebensmitteltechnologie und -Verfahrenstechnik (1982) 33(3), S. 139 ff. beschriebenen "Kratz"-Wärmetauscher, nach dem Rotor-Stator-Prinzip arbeitende Zerkleinerungsmaschinen, wie z.B. Zahnkranzdispergiermaschinen (z. B. vom Typ Megatron® der Fa. Kinematica), Kolloid- und Korundscheibenmühlen sowie Hochdruck- und Ultraschallhomogenisatoren.

Geeignete statische Inline-Mischer sind z. B. die in ZFL-Zeitschrift für Lebensmitteltechnologie und -Verfahrenstechnik (1982) 33(3) S. 139 ff. beschrieben, wie z. B. Ross-ISG-Mischer, bei denen der Fluidstrom durch Einbauten mit Bohrungen geführt wird, die ihn in Teilströme zerlegen, die anschließend seitlich versetzt und in anderer Reihenfolge wieder zusammengeführt werden. Geeignet sind weiterhin statische Mischer, die mehrere gleichartige oder verschiedene feststehende Mischelemente umfassen, die, z. B. jeweils versetzt, hintereinander in ein Rohr oder einen Kanal eingebaut sind. Dazu zählen z. B. Kenics-, Sulzer SMV- und Sulzer SMX-Mischer.

Weitere geeignete statische Inline-Mischer sind Scherspaltmischer, wie die in der EP-B-101 007 beschriebenen Strahldispergatoren.

Weitere geeignete Mischer sind auch Vorrichtungen zur Inline-Emulgierung, wie Membranen und Jet-Mischer.

In einer geeigneten Ausführung wird als Mischvorrichtung mindestens ein Inline-Mischer verwendet, der zweckmäßigerweise unmittelbar vor dem Reaktionsgefäß angebracht ist.

Besonders bevorzugt umfasst die Mischvorrichtung einen dynamischen Mischer und gegebenenfalls einen statischen Mischer. Werden zwei Mischer verwendet, so sind diese in Reihe geschaltet. Als dynamischer Mischer wird dabei vorzugsweise ein Rohrdurchlaufmischer oder eine Zahnkranzdispergiermaschine verwendet.

Geeignete Reaktionszonen für die Emulsionspolymerisation sind die dafür üblichen Reaktortypen. Dazu zählen z. B. Rührkessel. Geeignete Rührertypen umfassen z. B. Propellerrührer, Impellerrührer, Scheibenrührer, Blattrührer, Ankerrührer, Schrägblattrührer, Kreuzbalkenrührer, Wendelrührer, Schneckenrührer, etc.

Vorzugsweise werden vor Beginn der radikalischen Emulsionspolymerisation alle mit der wässrigen Polymerdispersion Pd) in Kontakt kommenden Anlagenteile mit einer Base in Kontakt gebracht. Geeignet sind dazu wässrige Lösungen der eingangs genannten Basen, vorzugsweise wässrige NaOH und KOH. Bevorzugt kommen verdünnte wässrige Lösungen (z. B. 2 bis 4 gew.-%ige) zum Einsatz. Dazu kann der Reaktor, einschließlich etwaiger Reaktoreinbauten, Rührvorrichtungen, Heiz-/Kühlvorrichtungen, etc., und alle mit dem Reaktoraustrag in Kontakt kommenden Oberflächen mit einer wässrigen Base gespült werden.

Die bei der Polymerisation entstandenen Dispersionen können im Anschluss an den Polymerisationsprozess einer physikalischen oder chemischen Nachbehandlung unterworfen werden. Solche Verfahren sind beispielsweise die bekannten Verfahren zur Restmonomerenreduzierung, wie z. B. die Nachbehandlung durch Zusatz von Polymerisationsinitiatoren oder Mischungen mehrerer Polymerisationsinitiatoren bei geeigneten Temperaturen, eine Nachbehandlung der Polymerlösung mittels Wasserdampf oder Strippen mit Inertgas oder Behandeln der Reaktionsmischung mit oxidierenden oder reduzierenden Reagenzien, Adsorptionsverfahren wie die Adsorption von Verunreinigung an ausgewählten Medien wie z. B. Aktivkohle oder eine Ultrafiltration. In einer bevorzugten Ausführungsform wird die erhaltene Polymerdispersion Pd) einer Ultrafiltration unterzogen. So gelingt es Dispersionen mit geringen Restmonomergehalten zu erzielen, wie sie insbesondere für pharmazeutische Anwendungen erforderlich sind. Durch Ultrafiltration gelingt es dabei insbesondere, Dispersionen Pd) mit einem sehr geringen Gehalt an polymerisierbaren Monomeren zu erhalten.

Zur Reinigung der Dispersion durch Ultrafiltration d.h. zur Abtrennung von im Dispergiermedium gelösten niedermolekularen Verbindungen wird die Dispersion unter Druck mit einer Membran in Kontakt gebracht und polymerfreies, die gelösten Verbindungen enthaltenes Permeat (Filtrat) auf der Rückseite der Membran bei einem geringeren Druck als auf der Feedseite abgezogen. Die durch Ultrafiltration abtrennbaren niedermolekularen Verbindungen sind ausgewählt unter Edukten, Zersetzungsprodukten der Edukte, Oligomeren, Salzen, Tenside, etc. und Mischungen davon. Als Retentat erhält man eine aufkonzentrierte Polymerdispersion, die an gelösten Verbindungen abgereichert ist. Damit die Polymerkonzentration nicht zu hoch ansteigt, kann die abgetrennte Menge an Permeat kontinuierlich oder diskontinuierlich im Retentat durch Dispergiermittel (Wasser) ergänzt werden. Eine Ultrafiltration, die nicht bis zur Trockene durchgeführt wird, sondern bei der die abgetrennte Menge an Permeat ergänzt wird, wird auch als Diafiltration bezeichnet. Im Allgemeinen kann die Dispersion in der Konzentration, in der sie in der Synthese anfällt, zur Ultrafiltration eingesetzt werden (z. B. 20 bis 40%ig in Wasser). Es ist selbstverständlich ebenfalls möglich, die Dispersion vor der Ultrafiltration mit Wasser zu verdünnen, diese dann bei einer niedrigeren Polymerkonzentration der Ultrafiltration zu unterziehen (vorzugsweise einer Diafiltration, wobei abgetrenntes Permeat durch Wasserzufuhr ins Retentat so ergänzt wird, dass die Polymerkonzentration konstant bleibt) und gegebenenfalls anschließend auf die gewünschte Polymerkonzentration aufzukonzentrieren.

Eine Verhinderung oder Minimierung der Ablagerung (Deckschichtaufbau) von Polymerteilchen auf der Membranoberfläche, die zu einer deutlichen Abnahme des Permeatflusses führt, kann z. B. durch Umpumpen, mechanische Bewegung der Membran oder Rühraggregate zwischen den Membranen erfolgen. Dabei wird vorzugsweise eine Relativgeschwindigkeit zwischen Membran und Dispersion im Bereich von 0,1 bis 20 m/s (Wickelgeometrie: 0,1 bis 5 m/s, Rohrgeometrie: 1 bis 6 m/s, Rotationsgeometrie: 2 bis 20 m/s) erzeugt. Die erfindungsgemäßen Polymerdispersionen weisen überraschenderweise eine dazu ausreichende Scherstabilität auf.

Bevorzugte Geometrien sind neben der Rohrmembrangeometrie mit Membraninnendurchmessern von 1 bis 25 mm, bevorzugt 2 bis 15 mm, eine Modulgeometrie, bei der die Scherung und der Transmembrandruck unabhängig voneinander eingestellt werden können, da in diesem Fall höhere Permeatflüsse erzielt werden können als mit Rohrmembranen.

Bei entsprechenden Modulen wird zum einen entweder durch mechanische Bewegung der Membran oder durch Rühraggregate zwischen den Membranen eine Relativgeschwindigkeit zwischen Membran und Suspension zwischen 2 bis 20 m/s erzeugt und zum anderen durch Steuerung der Feed-, Retentat- oder Permeatmenge der Transmembrandruck geregelt.

Die Diafiltration und Aufkonzentration kann in Batchfahrweise durch mehrmaligen Durchgang der Suspension durch die Membranmodule oder kontinuierlich durch einmaligen Durchgang durch eine oder mehrere nacheinander geschaltete Feed- und Bleedstufen erfolgen.

Für das Membranverfahren können Membrantrennschichten mit Porendurchmessern zwischen 1 nm (molekulare Trenngrenzen ca. 1 kD) und 1 $\mu$m, bevorzugt 10 nm (molekulare Trenngrenzen ca. 20 kD) bis 400 nm (molekulare Trenngrenzen ca. 500 kD) eingesetzt werden. Die Trennschichten können aus organischen Polymeren, Keramik, Metall, Kohlenstoff oder Kombinationen daraus bestehen und müssen in dem Feedmedium, bei der Filtrationstemperatur stabil sein. Aus mechanischen Gründen sind die Trennschichten in der Regel auf einer ein- oder mehrschichtigen porösen Unterstruktur aus dem gleichen oder auch mehreren unterschiedlichen Materialien wie die Trennschicht aufgebracht. Beispiele für mögliche Materialkombinationen sind in der folgenden Tabelle aufgeführt:

| Trennschicht | Unterstruktur (gröber als Trennschicht) |
| --- | --- |
| Metall | Metall |
| Keramik | Metall, Keramik oder Kohlenstoff |
| Polymer | Polymer, Metall, Keramik oder Keramik auf Metall |
| Kohlenstoff | Kohlenstoff, Metall oder Keramik |
| Keramik: z. B. $\alpha$-Al$_2$O$_3$, ZrO$_2$, TiO$_2$, SiC, gemischte keramische Werkstoffe<br>Polymer: z. B. PP, PTFE, PVDF, Polysulfon, Polyethersulfon, Polyetheretherketon, Polyamid, Polyacrylnitril, Regeneratcellulose | |

Besonders bevorzugt sind Trennschichten aus hydrophilen Materialien, wie z. B. Metall, Keramik, Regeneratcellulose, Acrylnitril und hydrophilisiertem Acrylnitril, Polysulfon und hydrophilisiertem Polysulfon, Polyethersulfon und hydrophilisiertem Polyethersulfon, Polyetheretherketon und hydrophilisiertem Polyetheretherketon sowie hydrophilisiertem PVDF. Beispiele wurden mit Regeneratcellulose (30 kD), hydrophilisiertem PVDF (30 nm) und hydrophilisiertem Polyethersulfon (20 kD) gefahren.

Die Membranen können prinzipiell in Flach-, Rohr-, Multikanalelement-, Kapillar- oder Wickelgeometrie eingesetzt werden, für die entsprechende Druckgehäuse, die eine Trennung zwischen Retentat und dem Permeat erlauben, verfügbar sind.

Die optimalen transmembranen Drücke zwischen Retentat und Permeat liegen im Wesentlichen abhängig von dem Durchmesser der Membranporen, den hydrodynamischen Bedingungen, die den Deckschichtaufbau beeinflussen, und der mechanischen Stabilität der Membran bei der Filtrationstemperatur je nach Membranart vorzugsweise in einem Bereich von 0,2 bis 20 bar, besonders bevorzugt in einem Bereich von 0,3 bis 6 bar. Höhere transmembrane Drücke führen in der Regel zu höheren Permeatflüssen. Dabei kann in dem Fall, in dem mehrere Module in Serie geschaltet sind, der Transmembrandruck für jeden Modul durch Anhebung des Permeatdruckes abgesenkt und damit angepasst werden. Die Betriebstemperatur ist abhängig von der Membranstabilität und der Temperaturstabilität der Dispersion. Ein geeigneter Temperaturbereich für die Ultrafiltration ist 20 bis 80 °C. Höhere Temperaturen führen in der Regel zu höheren Permeatflüssen. Die erreichbaren Permeatflüsse sind stark von der eingesetzten Membranart und Membrangeometrie, von den Prozessbedingungen, von der Feedzusammensetzung (im Wesentlichen der Polymerkonzentration) abhängig. Die Flüsse liegen typischerweise in einem Bereich von 5 bis 500 kg/m$^2$/h. So werden z. B. in einem Rotationssystem bei 30 % Polymergehalt, 40 °C und einem Transmembrandruck von 1 bar an einer Regeneratcellulosemembran mit einer Trenngrenze von 30 kD je nach Rotationsgeschwindigkeit Permeatflüsse in einem Bereich von 15 bis 70

kg/m$^2$/h erreicht. Dabei steigt der Permeatfluss typischerweise vom Start der Ultrafiltration bis zum Ende ca. um den Faktor 2. Die gewünschte Abreicherung der wasserlöslichen Inhaltsstoffe wird durch einen Diafiltrationsschritt mit einem Lösungsmittelaustauschkoeffizienten von bis zu 5, vorzugsweise von bis zu 3, im Allgemeinen erreicht. Dabei gibt der Lösungsmittelaustauschkoeffizient an wie oft das Lösungsmittel der Dispersion während der Diafiltration ausgetauscht wird. D. h. bei einem Lösungsmittelaustauschkoeffizient von X wird pro m$^3$ Dispersion X m$^3$ Permeat abgetrennt und gleichzeitig X m$^3$ Lösungsmittel (Wasser) in die Dispersion zugeführt.

Folgende Membranen sind z. B. einsetzbar:

| Hersteller | Membran | Trenngrenze (kD) Porendurchmesser (nm) |
|---|---|---|
| Atech innovations GmbH | UF / TiO$_2$ auf $\alpha$-Al$_2$O$_3$ / 1, 2 | 20 kD |
| | UF / ZrO$_2$ auf $\alpha$-Al$_2$O$_3$ / 1, 2 | 50 nm |
| | MF / $\alpha$-Al$_2$O$_3$ auf $\alpha$-Al$_2$O$_3$ / 1, 2 | 0,1; 0,2; 0,4 $\mu$m |
| Rhodia / Orelis | MF / ZrO$_2$ oder TiO$_2$ auf Keramik / 1, 2 | 0,1; 0,2; 0,45 $\mu$m |
| | UF / ZrO$_2$ oder TiO$_2$ auf Keramik / 1, 2 | 15, 50, 150; 300 kD |
| | UF / ZrO$_2$TiO$_2$ auf Kohlenstoff / 1 | 50; 150; 300 kD |
| | MF / ZrO$_2$-TiO$_2$ auf Kohlenstoff / 1 | 0,14 $\mu$m |
| Pall-Schumacher | UF / TiO$_2$ oder ZrO$_2$ auf Keramik / 1, 2 | 5,10 und 50 nm |
| | MF / $\alpha$-Al$_2$O$_3$ auf Keramik | 100 und 200 nm |
| Graver Technologies | UF / TiO$_2$auf Stahl / 1 | 100 nm |
| Creavis | UF / ZrO$_2$ auf $\alpha$-Al$_2$O$_3$ und Metall / 3 | 25, 80 nm |
| Bekaert | MF / Metall auf Metall / 1 | 0,2 - 0,5 $\mu$m |
| GKN | MF / Metall auf Metall / 1 | 0,3 $\mu$m |
| NADIR Filtrations GmbH | UF / Polyethersulfon (hydrophilisiert) / 1,3 | 4 - 150 kD |
| | UF / PAN / 1 | 20, 40 kD |
| | U F / Regeneratcellulose/ 3 | 5, 10, 30, 100 kD |
| Osmonics / Desal | UF / PAN (hydrophilisiert) / 3 | 100 kD |
| X-Flow | UF / PVDF (hydrophilisiert) /1 | 30 nm |
| 1: Rohrmembran; 2: Mehrkanalelement; 3: Flachmembran für Wickel-, Taschen-, Plattenstapel- oder Sondermodule mit bewegter Membran bzw. Rühraggregaten zwischen den Membranen | | |

Die erfindungsgemäßen Polymerdispersionen Pd) zeichnen sich, nachdem sie einer Ultrafiltration unterzogen wurden, durch sehr geringe Restgehalte an im Dispergiermedium gelösten niedermolekularen Verbindungen aus. Dies gilt insbesondere für unerwünschte Hydrolyseprodukte aus den Monomeren a) und b). So gelingt es Dispersionen bereit zu stellen, die sich insbesondere als Überzugsmittel für pharmazeutische Darreichungsformen eignen.

Die erfindungsgemäßen Polymerdispersionen Pd) enthalten N,N-Diethylaminoethanol vorzugsweise in einer Menge von höchstens 2500 Gew.-ppm, besonders bevorzugt von höchstens 1000 Gew.-ppm, insbesondere von höchstens 500 Gew.-ppm.

Die erfindungsgemäßen Polymerdispersionen Pd) enthalten Methacrylsäure vorzugsweise in einer Menge von höchstens 100 Gew.-ppm, besonders bevorzugt von höchstens 50 Gew.-ppm.

Die erfindungsgemäßen Polymerdispersionen Pd) enthalten Methanol vorzugsweise in einer Menge von höchstens 500 Gew.-ppm, besonders bevorzugt von höchstens 250 Gew.-ppm, insbesondere von höchstens 50 Gew.-ppm.

Es ist prinzipiell möglich, den erfindungsgemäßen Polymerdispersionen Pd) wenigstens einen der zuvor genannten Zusatz- oder Hilfsstoffe während oder nach der Ultrafiltration zuzusetzen. Dazu zählen z. B. Puffersubstanzen, Konservierungsmittel oder Antioxidantien.

Es können sich auch weitere Aufarbeitungsschritte anschließen, beispielsweise geeignete Trockenverfahren. Die Polymerdispersionen Pd) können durch verschiedene Trocknungsverfahren, wie z. B. Sprühtrocknung, Fluidized

EP 2 176 301 B1

Spray Drying, Walzentrocknung oder Gefriertrocknung in Pulverform überführt werden. Bevorzugt wird die Sprühtrocknung eingesetzt. Die so erhaltenen Polymer-Trockenpulver lassen sich vorteilhafterweise durch Redispergieren in Wasser bei einem geeigneten pH-Wert erneut in eine wässrige Dispersion überführen. Bevorzugt werden die nach dem erfindungsgemäßen Verfahren erhaltenen Dispersionen jedoch direkt als Primärdispersion in den Handel gebracht und/ oder weiterverarbeitet.

Überraschenderweise besitzen die erfindungsgemäßen Dispersionen eine hervorragende physikalische und chemische Stabilität. So sedimentieren oder koagulieren die Dispersionen bei Lagerung über mehrere Jahre bei Raumtemperatur nicht. Die anwendungstechnischen Eigenschaften sind nach Lagerung unverändert. Entgegen aller Erwartung findet nur eine minimale Verseifung der Estergruppen des Polymers statt und dies trotz eines alkalischen pH-Wertes und der Anwesenheit von Aminogruppen, die die Verseifung üblicherweise katalysieren.

Die in den erfindungsgemäßen Dispersionen Pd) enthaltenen Polymere weisen vorzugsweise ein mittels Gelpermeationschromatographie bestimmtes mittleres Molekulargewicht $M_w$ im Bereich von 30000 bis 500000, besonders bevorzugt 60000 bis 140000, insbesondere 80000 bis 120000 g/mol, auf.

Die Durchführung der GPC erfolgt nach folgender Methode:

| | |
|---|---|
| Elutionsmittel: | THF + 0,02 mol/l Triethylamin |
| Säulen-Temperatur: | 35 °C |
| Durchflussgeschw.: | 1 mL/min |
| Injektion: | 100 $\mu$L einer Lösung mit 2 [g/l] |

Probelösungen wurden über Sartorius Minisart SRP 25 (Porenweite 0,2 [$\mu$m]) filtriert.

Trennsäulen-Kombination:

| Nr. | Säulen i. D. [mm] | Länge [cm] | Trennmaterial | Ausschlussgrenze |
|---|---|---|---|---|
| 775 | 7,5 | 30 | PL Gel Mixed B | 8000000 |
| 776 | 7,5 | 30 | PL Gel Mixed B | 8000000 |

Bödenzahl der Kombination bei der angegebenen Durchflussgeschwindigkeit: 40000

Detektor:     Differentialrefraktometer HP-1100

Kalibrierung:     Die Kalibrierung erfolgte mit eng verteilten Polystyrol-Standards der Fa. Polymer Laboratories mit Molekulargewichten von M = 580 bis M = 7500000, sowie Hexylbenzol (M = 162). Außerhalb dieses Intervalls liegende Elutionsbereiche wurden durch Extrapolation geschätzt.

Die in den erfindungsgemäßen Dispersionen Pd) enthaltenen Polymere weisen vorzugsweise einen K-Wert (bestimmt nach Fikentscher an einer 1 %igen Lösung in N-Methylpyrrolidon (NMP)) im Bereich von 40 bis 60 auf.

Bei der Herstellung 1 %iger Lösungen zur K-Wertbestimmung wird eine Probenmenge, deren Feststoffanteil etwa 0,7 g beträgt, eingewogen und mit dem berechneten Volumen an Lösungsmittel versetzt. Der Anteil des Lösungsmittels berechnet sich nach folgender Gleichung:

$$V_L = \left( \frac{m_P \, * \, FG}{c} \right) - m_P$$

Hierbei bedeuten:

$V_L$     = berechnetes Lösungsmittelvolumen [ml]

$m_P$    = Probeneinwaage [g]

FG    = Feststoffgehalt [g/100 g]

c    = Sollkonzentration [g/100 ml]

Die Dichte der Probe wird bei dieser Berechnung als 1 g/ml angenommen. Das Gesamtvolumen liegt bei ungefähr 70 ml. Die Lösung wird bei Raumtemperatur gelöst und durch ein 0,40 $\mu$m Metallsieb filtriert. Mit den filtrierten Lösungen und reinem Lösungsmittel wird dann eine Viskositätsmessung durch Bestimmung der Durchlaufzeiten mit einem Kapillarviskosimeter bei 25 °C ($\pm$0,05 °C) durchgeführt.

Die Glasübergangstemperatur $T_G$ (bestimmt mittels DSC) liegt vorzugsweise in einem Bereich von 40 bis 70 °C, besonders bevorzugt 52 bis 62 °C.

Bei den in den erfindungsgemäßen Dispersionen Pd) enthaltenen Polymeren handelt es sich im Wesentlichen um statistische Copolymere.

Der mittlere Teilchendurchmesser der in der Polymerdispersion Pd) enthaltenen Polymerteilchen (bestimmt mittels analytischer Ultrazentrifuge) liegt vorzugsweise in einem Bereich von 70 bis 200 nm, besonders bevorzugt von 80 bis 150 nm, insbesondere von 90 bis 120 nm Die Teilchengrößenverteilung ist vorzugsweise im Wesentlichen unimodal.

Der LD-Wert der erfindungsgemäßen Dispersionen Pd), bestimmt an einer 0,01 %igen Dispersion in Wasser (2,5 cm Küvette, weißes Licht) beträgt vorzugsweise wenigstens 70 %, besonders bevorzugt wenigstens 80 %. Die Bestimmung der Lichtdurchlässigkeit wird z. B. in Dieter Distler, Wässrige Polymerdispersionen, Wiley-VCH (1999), S. 40, beschrieben.

Der Feststoffgehalt der erfindungsgemäßen Dispersionen Pd) beträgt vorzugsweise 10 bis 50 Gew.-%, besonders bevorzugt 20 bis 40 Gew.-%. Im Falle einer Reinigung der Dispersion mittels Ultrafiltration weisen die erfindungsgemäßen Dispersionen vorzugsweise vor und nach der Ultrafiltration Feststoffgehalte auf, die in diesen Bereichen liegen. Selbstverständlich ist es ebenfalls möglich, eine verdünnte Polymerdispersion einer Aufkonzentrierung durch Ultrafiltration zu unterziehen.

Die erfindungsgemäßen Dispersionen weisen z. B. auch bei einem Feststoffgehalt von 30 Gew.-% extrem niedrige Viskositäten von vorzugsweise kleiner 50 mPas, besonders bevorzugt kleiner 25 mPas und insbesondere kleiner 10 mPas auf (Werte bestimmt mittels Brookfield-Viskosimeter bei 20 °C und 100 s$^{-1}$). Solche niedrigen Viskositäten sind für viele Anwendungen von besonderer Bedeutung.

Die Ladung der in den erfindungsgemäßen Dispersionen Pd) enthaltenen Polymere ist abhängig vom pH-Wert der Dispersion. Der isoelektrische Punkt liegt vorzugsweise in einem pH-Bereich von etwa 7,5 bis 8,5. Die fertige Dispersion weist vorzugsweise einen pH-Wert im Bereich von 8 bis 10, besonders bevorzugt von 8,5 bis 9,5 (bei einem Feststoffgehalt von 30 Gew.-%), auf. Es ist vorteilhaft, dass der pH-Wert der fertigen Dispersion höher (stärker alkalisch) gewählt wird als ihr isoelektrischer Punkt, solange eine Auflösung oder Quellung der in der Dispersion enthaltenen Polymerteilchen nicht gewünscht ist. Bei den erfindungsgemäßen Dispersionen handelt es sich deshalb vorzugsweise um basische Dispersionen.

Die erfindungsgemäßen Polymerdispersionen Pd) zeichnen sich durch ihre pH-abhängige Löslichkeit aus. Eine Einstellung des pH-Bereichs, in dem sich die Dispersion beim Ansäuern auflöst gelingt z. B. durch die einpolymerisierte Menge an N,N-Diethylaminoethylmethacrylat (Monomer a) sowie gegebenenfalls den Einsatz weiterer Monomere mit kationogener/kationischer Gruppen (Monomer d). Vorzugsweise lösen sich die in den erfindungsgemäßen Polymerdispersionen Pd) enthaltenen Polymere bei einem pH von höchstens 6,8, besonders bevorzugt bei einem pH von höchstens 5,5.

Ein weiterer Gegenstand der Erfindung ist daher eine wässrige, durch Verringerung des pH-Werts lösliche Polymerdispersion Pd), enthaltend

- wenigstens ein Polymer, das

 a) N,N-Diethylaminoethylmethacrylat, und

 b) wenigstens eine radikalisch polymerisierbare Verbindung, ausgewählt unter Estern $\alpha,\beta$-ethylenisch ungesättigter Mono- und Dicarbonsäuren mit $C_1$-$C_8$-Alkanolen,

 einpolymerisiert enthält,

- wenigstens einen Emulgator, ausgewählt unter anionischen und nichtionischen Emulgatoren, und

- Wasser.

Eine bevorzugte Ausführung sind solche Polymerdispersionen, die ein Polymer enthalten, das

- 43 bis 47 Gew.-%, bezogen auf das Gesamtgewicht der zur Polymerisation eingesetzten Monomere, N,N-Diethylaminoethylmethacrylat a), und

- 53 bis 57 Gew.- %, bezogen auf das Gesamtgewicht der zur Polymerisation eingesetzten Monomere, wenigstens einer Verbindung b)

als einzige Monomere einpolymerisiert enthält.

Die zuvor beschriebenen Polymerdispersionen Pd) eignen sich hervorragend zur Herstellung pharmazeutischer Mittel. Mit ihnen wird erstmalig eine wässrige Dispersion eines basischen aminogruppenhaltigen Polymers bereitgestellt, die für den pharmazeutischen Einsatz geeignet ist, eine entsprechende Reinheit aufweist und über eine ausreichende Stabilität verfügt. Sie dienen dabei z. B. als polymere Filmbildner, insbesondere zur Herstellung von Überzugsmitteln für pharmazeutische Darreichungsformen.

Die Formulierungsgrundlage für erfindungsgemäße Überzugsmittel für pharmazeutische Darreichungsformen kann wenigstens einen weiteren pharmazeutisch akzeptablen Hilfsstoff enthalten. Pharmazeutisch akzeptabel sind die im Bereich der Pharmazie, der Lebensmitteltechnologie und angrenzenden Gebieten bekanntermaßen verwendbaren Hilfsstoffe, insbesondere die in einschlägigen Arzneibüchern (z. B. Ph. Eur., USP, JP) gelisteten sowie andere Hilfsstoffe, deren Eigenschaften einer physiologischen Anwendung nicht entgegenstehen.

Geeignete Hilfsstoffe können sein: Aromastoffe, geschmacksverbessernde Stoffe, Süßungsmittel (Zucker, Zuckeralkohole, Süßstoffe wie z. B. Aspartame, Saccharin-Na, Natriumcyclamat), Gleitmittel, Netzmittel, Trennmittel, Weichmacher, Antiklebemittel, Antioxidantien, Stabilisatoren, Porenbildner, Neutralisierungsmittel, Glanzmittel, Farbstoffe, Pigmente, Desinfektions- oder Konservierungsmittel, Verdickungsmittel, etc. Solche Stoffe sind z. B. in Fiedler, H. P. Lexikon der Hilfsstoffe für Pharmazie, Kosmetik und angrenzende Gebiete, 4. Aufl., Aulendorf: ECV-Editio-Cantor-Verlag, 1996, beschrieben.

Übliche Mengen der Hilfsstoffe liegen in einem Bereich von jeweils 0 bis 50 Gew.-%, bevorzugt 0 bis 20 Gew.-%, insbesondere 0,01 bis 10 Gew.-%, bezogen auf das Gesamtgewicht des Überzugsmittels.

Die Herstellung der Überzugsmittel kann z. B. durch inniges Vermischen einer erfindungsgemäßen Polymerdispersion oder eines daraus durch ein Trocknungsverfahren erhältlichen Polymers mit wenigstens einem Hiffsstoff erfolgen. In einer besonders bevorzugten Ausführungsform wird die erfindungsgemäße Polymerdispersion als solche, ohne vorherige Trocknung, zur Herstellung eines Überzugsmittels eingesetzt. Möglich ist jedoch auch, eine erfindungsgemäße Polymerdispersion zunächst einer Trocknung zu unterziehen und die so erhaltene Polymerzusammensetzung zur Herstellung eines Überzugsmittels einzusetzen. Die Polymerzusammensetzung kann dann z. B. in Pulverform, als Schmelze, Lösung oder Sekundärdispersion zur Herstellung des Überzugsmittels eingesetzt werden.

Ein weiterer Gegenstand der Erfindung ist ein Überzugsmittel, enthaltend eine solche wässrige Polymerdispersion Pd) oder eine daraus durch Trocknung erhältliche Polymerzusammensetzung.

Ein weiterer Gegenstand der Erfindung ist ein pharmazeutisches Mittel oder Lebensmittel, enthaltend

A) eine Polymerzusammensetzung, erhältlich durch Trocknen und/oder Verfilmen einer solchen Polymerdispersion,

B) wenigstens einen pharmazeutisch akzeptablen Wirkstoff oder einen Nahrungsergänzungsstoff und

C) gegebenenfalls wenigstens einen pharmazeutisch oder im Lebensmittelbereich akzeptabien Hilfsstoff.

Eine spezielle Ausführungsform ist ein pharmazeutisches Mittel oder Lebensmittel in Form einer oralen Darreichungsform, umfassend einen Überzug auf Basis einer solchen wässrigen Polymerdispersion Pd) oder einer daraus durch Trocknung erhältlichen Polymerzusammensetzung.

Die erfindungsgemäßen Polymerdispersionen können in den pharmazeutischen Mitteln z. B, zur Herstellung eines Bindemittels oder eines Überzugsmittels dienen. Die funktionelle Wirkung tritt dabei in der Regel durch Trocknung und/oder Verfilmung des Überzugs- oder Bindemittels ein. Diese Trocknung und/oder Verfilmung kann, unabhängig von dem Auftragsverfahren, durch Zuführung von Energie erfolgen. Dies kann über Konvektion (Wärme), Strahlung (Infrarot oder Mikrowellen) oder Leitung erfolgen. Für den Auftrag als Dispergiermittel eingesetztes Wasser verdampft dabei, gegebenenfalls kann auch ein Vakuum angewendet werden, um das Verdampfen zu beschleunlgen.

Das erfindungsgemäße Überzugsmittel kann z. B, in Pulverform, als Schmelze oder in wässriger Emulsion durch Granulieren, Gießen. Ausstreichen oder mittels sprühauftrag angewendet werden. Bevorzugt ist die Anwendung als Polymerdispersion, speziell als Primärdispersion. Die erfindungsgemäßen Überzugsmittel können zusätzlich wenigstens eine weitere Polymerkomponente enthalten. Dabei können Mischungen aus Wenigstens zwei Dispersionen, wenigstens einer Dispersion und wenigstens einer Lösung, wenigstens einer Dispersion und wenigstens einem Pulver, wenigstens zwei Pulvern, etc. zum Einsatz kommen.

Die erfindungsgemäße Formulierung eignet sich zur Verabreichung grundsätzlich beliebiger pharmazeutischer Wirkstoffe, die vorzugsweise in isolierter oder geschützter Form verabreicht werden können, wie Antidepressiva, Betarezeptorenblocker, Antidiabetika, Analgetika, Antiphlogistika, Antirheumatika, Antihypotonika, Antihypertonika, Psychopharmaka, Tranquilizer, Antiemetika, Muskelrelaxantien, Glucocorticoide, Mittel zur Behandlung von Colitis ulcerosa oder Morbus Crohn, Antiallergika, Antibiotika, Antiepileptika, Antikoagulantia, Antimykotika, Antitussiva, Arteriosklerosemittel, Diuretika, Enzyme, Enzyminhibitoren, Gichtmittel, Hormone und deren Hemmstoffe, Herzglykoside, Immuntherapeutika und Zytokine, Laxantien, Lipidsenker, Magen- Darmtherapeutika, Migränemittel, Mineralstoffpräparate, Otologika, Parkinsonmittel, Schilddrüsentherapeutika, Spasmolytika, Thrombozytenaggregationshemmer, Vitamine, Zytostatika und Metastasenhemmer, Phytopharmaka, Chemotherapeutika, Nutraceuticals, Vitamine, Carotinoide und Aminosäuren.

Beispiele geeigneter Wirkstoffe sind: Acarbose, Nichtsteroidale Antirheumatika, Herzglykoside, Acetylsalicylsäure, Virustatika, Aclarubicin, Aciclovir, Cisplatin, Actinomycin, $\alpha$-und $\beta$-Sympatomimetika, Allopurinol, Alosetron, Alprostadil, Prostaglandine, Amantädin, Ambroxol, Amlodipin, Methotrexat, 5-Aminosalicylsäure, Amitriptylin, Amlodipin, Amoxicillin, Anastrozol, Atenolol, Atorvastatin, Azathioprin, Balsalazid, Beclomethason, Betahistin, Bezafibrat, Bicalutamid, Diazepam und Diazepamderivate, Budesonid, Bufexamac, Buprenorphin, Methadon, Calciumsalze, Kaliumsalze, Magnesiumsalze, Candesartan, Carbamazepin, Captopril, Cefalosporine, Celetoxib, Cetirizin, Chenodeoxycholsäure, Ursodeoxycholsäure, Theophyllin und Theophyllinderivate, Trypsine, Cimetidin, Clarithromycin, Clavulansäure, Clindamycin, Clobutinol, Clonidin, Cotrimoxazol, Codein, Coffein, Vitamin D und. Derivate von Vitamin D, Colestyramin, Cromoglicinsäure, Cumarin und Cumarinderivate, Cystein, Cytarabin, Cyclophosphamid, Ciclosporin, Cyproteron, Cytarabin, Dapiprazol, Desogestrel, Desonid, Dihydralazin, Diltiazem, Mutterkornalkaloide, Dimenhydrinat, Dimethylsulfoxid, Dimeticon, Dipyridamol, Domperidon und Domperidonderivate, Donepzil, Dopamin, Doxazosin, Doxorubicin, Doxylamin, Dapiprazol, Benzodiazepine, Diclofenac, Glykosidantibiotika, Desipramin, Econazol, ACE-Hemmer, Enalapril, Ephedrin, Epinephrin, Epoetin und Epoetinderivate, Morphinane, Calciumantagonisten, Irinotecan, Modafinil, Orlistat, Peptidantibiotika, Phenytoin, Riluzole, Risedronat, Sildenafil, Topiramat, Makrolidantibiotika, Esomeprazol, Estrogen und Estrogenderivate, Gestagen und Gestagenderivate, Testosteron und Testosteronderivate, Androgen und Androgenderivate, Ethenzamid, Etofenamat, Etofibrat, Fenofibrat, Etofyllin, Etoposid, Famciclovir, Famotidin, Felodipin, Fenofibrat, Fentanyl, Fenticonazol, Gyrasehemmer, Fluconazol, Fludarabin, Flunarizin, Fluorouracil, Fluoxetin, Flurbiprofen, Ibuprofen, Flutamid, Fluvastatin, Follitropin, Formoterol, Fosfomicin, Furosemid, Fusidinsäure, Galantamin, Gallopamil, Ganciclovir, Gemfibrozil, Gentamicin, Ginkgo, Johanniskraut, Glibenclamid, Harnstoffderivate als orale Antidiabetika, Glucagon, Glucosamin und Glucosaminderivate, Glutathion, Glycerol und Glycerofderivate, Hypothalamushormone, Goserelin, Guanethidin, Halofantrin, Haloperidol, Heparin und Heparinderivate, Hyaluronsäure, Hydralazin, Hydrochlorothiazid und Hydrochlorothiazidderivate, Salicylate, Hydroxyzin, Idarubicin, Ifosfamid, Imipramin, Indometacin, Indoramin, Insulin, Interferone, Jod und Jodderivate, Isoconazol, Isoprenalin, Glucitol und Glucitolclerivate, Itraconazol, Ketoconazol, Ketoprofen, Ketotifen, Lacidipin, Lansoprazol, Levodopa, Levomethadon, Schilddrüsenhormone, Liponsäure und Liponsäurederivate, Lisinopril, Lisurid, Lofepramin, Lomustin, Loperamid, Loratadin, Maprotilin, Mebendazol, Mebeverin, Meclozin, Mefenaminsäure, Mefloquin, Meloxicam, Mepindolol, Meprobamat, Meropenem, Mesalazin, Mesuximid, Metamizol, Metformin, Methotrexat, Methylphenidat, Methylprednisolon, Metixen, Metoclopramid, Metoprolol, Metronidazol, Mianserin, Miconazol, Minocyclin, Minoxidil, Misoprostol, Mitomycin, Mizolastin, Moexipril, Morphin und Morphinderivate ; Nachtkerze, Nalbuphin, Naloxon, Tilidin, Naproxen, Narcotin, Natamycin, Neostigmin, Nicergolin, Nicethamid, Nifedipin, Nifluminsäure, Nimodipin, Nimorazol, Nimustin, Nisoldipin, Adrenalin und Adrenalinderivate, Norfloxacin, Novaminsulfon, Noscapin, Nystatin, Ofloxacin, Olanzapin, Olsalazin, Omeprazol, Omoconazol, Ondansetron, Orlistat, Oseltamivir, Oxaceprol, Oxacillin, Oxiconazol, Oxymetazolin, Pantoprazol, Paracetamol, Paroxetin, Penciclovir, orale Penicilline, Pentazocin, Pentifyllin, Pentoxifyllin, Perphenazin, Pethidin, Pflanzenextrakte, Phenazon, Pheniramin, Barbitursäurederivate, Phenylbutazon, Phenytoin, Pimozid, Pindolol, Piperazin, Piracetam, Pirenzepin, Piribedil, Piroxicam, Pramipexol, Pravastatin, Prazosin, Procain, Promazin, Propiverin, Propranolol, Propyphenazon, Prostaglandine, Protionamid, Proxyphyllin, Quetiapin, Quinapril, Quinaprilat, Ramipril, Ranitidin, Reproterol, Reserpin, Ribavirin, Rifampicin, Risperidon, Ritonavir, Ropinirol, Rosiglitazon, Roxatidin, Roxithromycin, Ruscogenin, Rutosid und Rutosidderivate, Sabadilla, Salbutamol, Salmeterol, Scopolamin, Selegilin, Sertaconazol, Sertindol, Sertralin, Silikate, Simvastatin, Sitosterin, Sotalol, Spagluminsäure, Sparfloxacin, Spectinomycin, Spiramycin, Spirapril, Spironolacton, Stavudin, Streptomycin, Sucralfat, Sufentanil, Sulbactam, Sulfonamide, Sulfasalazin, Sulpirid, Sultamicillin, Sultiam, Sumatriptan, Suxamethoniumchlorid, Tacrin, Tacrolimus, Taliolol, Tamoxifen, Taurolidin, Tazaroten, Tegaserod, Temazepam, Teniposid, Tenoxicam, Terazosin, Terbinafin, Terbutalin, Terfenadin, Terlipressin, Tertatolol, Tetracycline, Tetryzolin, Theobromin, Theophyllin, Butizin, Thiamazol, Phenothiazine, Thiotepa, Tiagabin, Tiaprid, Propionsaurederivate, Ticlopidin, Timolol, Tinidazol, Tioconazol, Tioguanin, Tioxolon, Tiropramid, Tizanidin, Tolazolin, Tolbutamid, Tolcapon, Tolnaftat, Tolperison, Topotecan, Torasemid, Antiöstrogene, Tramadol, Tramazolin, Trandolapril, Tranylcypromin, Trapidil, Trazodon, Triamcinolon und Triamcinolonderivate, Triamteren, Trifluperidol, Trifluridin, Trimethoprim, Trimipramin, Tripelennamin, Triprolidin, Trifosfamid, Tromantadin, Trometamol, Tropalpin, Troxerutin, Tulobuterol, Tyramin, Tyrothricin, Urapidil, Ursodeoxycholsäure, Chenodeoxycholsäure, Valaciclovir, Valdecoxib, Valproinsäure, Vancomycin, Vecuroniumchlorid, Venlafaxin, Verapamil, Vidarabin, Vigabatrin, Viloxazin, Vinblastin, Vincamin, Vincristin, Vindesin, Vinorelbin, Vinpocetin, Viquidil, Warfarin, Xantinolnicotinat, Xipamid, Zafirlukast, Zalcitabin, Zanamivir, Zidovudin, Zolmitriptan, Zolpidem, Zopiclon, Zotepin und dergleichen.

Die Wirkstoffe können gewünschtenfalls auch in Form ihrer pharmazeutisch akzeptablen Salze oder Derivate verwendet werden, und im Falle chiraler Wirkstoffe können sowohl optisch aktive Isomere als auch Racemate oder Diastereoisomerengemische eingesetzt werden. Gewünschtenfalls können die erfindungsgemäßen Zusammensetzungen auch zwei oder mehrere pharmazeutische Wirkstoffe enthalten.

Die erfindungsgemäßen Dispersionen werden bevorzugt zum Überziehen von Tabletten, Extrudaten, Minitabletten, Kapseln, Weichkapseln, Granulaten, Pellets, Mikropellets, Mikrokapseln, Kristallen eingesetzt. Gecoatete Granulate, Pellets, Mikropellets, Mikrokapseln, Kristalle können mit geeigneten Hilfsstoffen abgemischt und zu Tabletten verpresst werden, die in Wasser schnell zerfallen und die gecoateten feinen Formlinge wieder freigeben. Auf diese Weise können so genannte MUPS-Formen, multiple unit particulate systems hergestellt werden. Dies sind Tabletten, die nach der Einnahme zerfallen und die mit einem erfindungsgemäßen Überzugsmittel überzogene Untereinheiten freisetzen. Besondere Bedeutung haben hierbei so genannte oral dispersibles, d. h. Tabletten, die im Mund innerhalb von kurzer Zeit zerfallen und geschmacksmaskierte kleine Formlinge freisetzen.

In einer speziellen Variante werden die erfindungsgemäßen Dispersionen zum Granulieren von Wirkstoffen gegebenenfalls mit entsprechenden Hilfsstoffen verwendet und die Granulate anschließend zu Tabletten verpresst.

Die erfindungsgemäßen Dispersionen können auch zur Herstellung von transdermalen Wirkstoffpflastern oder Sprays herangezogen werden.

Geeignet ist ebenfalls die Einbettung von Agglomeraten in Polyethylenglykol oder Lipide zur Herstellung von Suppositorien oder vaginalen Arzneiformen.

Wirkstoffklassen und Substanzen, die oftmals bitteren Geschmack hervorrufen können und sich vorteilhaft mit dem erfindungsgemäßen Überzugs- und Bindemittel formulieren lassen, sind z. B.:

Analgetika und Antirheumatika, wie Paracetamol, Diclofenac, Aceclofenac, Ibuprofen, Ketoprofen, Flubiprofen, Acetylsalicylsäure, Levacetylmethadol und Oxycodon;
Psychopharmaka, wie Promethazine, Donepezil, Modafinil, Nefazodon, Reboxetin, Sertindol und Sertralin;
Antibiotika, wie Erythromycin, Roxithromycin, Clarithromycin, Grepafloxacin, Ciprofloxacin, Levofloxacin, Sparfloxacin, Trovafloxacin und Nevirapin;
Betablocker, wie Propranolol, Metoprolol, Bisoprolol und Nebivolol;
Antidiabetika, wie Metformin, Miglitol und Repaglinid;
$H_1$-Antihistaminika, wie Diphenhydramin, Fexofenadin und Mizolastin;
$H_2$ Antihistaminika, wie Cimetidin, Famotidin, Roxatidin, Nizatidin, Ticlopidin, Cetirizin und Ranitidin;
Vitamine wie Thiaminnitrat sowie Chinidin-Sulfat, Amyloprilose-HCl, Pseudoephedrin-HCl, Sildenafil, Topiramat, Granisetron, Rebamipide, Chinin- HCl, etc.

Die hervorragende Eignung zur Geschmacksmaskierung resultiert aus der Unlöslichkeit der erfindungsgemäßen Polymeren bei pH-Werten größer 6 und der schnellen Löslichkeit bei pH-Werten unter 6. Das heißt im Speichel (pH-Wert: 7,2) sind entsprechend überzogene Formen sehr lange stabil und es erfolgt kein Kontakt des bitteren Arzneistoffs mit der Mundschleimhaut, aber im Magen bei pH-Werten von 1 bis 5 erfolgt eine sehr schnelle Freisetzung des Wirkstoffs. Die Auflösung ist dabei so schnell, dass kein Unterschied im Wirkungseintritt vorhanden ist, verglichen mit einer nicht gecoateten Form. In der Regel lösen sich Filmüberzüge eines erfindungsgemäßen Polymers innerhalb von 5 min in Magensaft auf, wohingegen sie in Phosphatpuffer pH 7,2 über 2 Stunden stabil sind. Überraschenderweise lösen sich die Filmüberzüge auch in Medien mit pH-Werten von 4,5 relativ schnell, so dass die daraus hergestellten Darreichungsformen auch bei anaciden Patienten bzw. Patienten, die mit Antacida behandelt werden, eine schnelle Wirkung entfalten.

Die erfindungsgemäßen Überzugsmittel weisen eine niedrige Wasserdampf- und Sauerstoffpermeabilität auf und ermöglichen dadurch die Formulierung und Stabilisierung besonders wasserdampfempfindlicher oder sauerstoffempfindlicher Arzneistoffe wie z. B. Acetylsalicylsäure, Enalapril, Cortisonacetat, Omeprazol, Carotinoide. Hierbei hat der Überzug protektiven Charakter.

Darüber hinaus können die erfindungsgemäßen Überzugsmittel zur Trennung inkompatibler Wirk- oder Hilfsstoffe in Darreichungsformen herangezogen werden, indem ein oder mehrere Bestandteile umhüllt werden und so der gegenseitige Kontakt vermieden wird.

Die unerwartet sehr guten anwendungstechnischen Eigenschaften der erfindungsgemäßen Filmüberzüge werden ermöglicht durch eine ausgezeichnete homogene Verfilmung der Polymerdispersion, eine geringe Klebrigkeit der Filme und die gute Flexibilität bzw. Dehnbarkeit der Überzüge, so dass auch bei Quellung des Tabletten- oder Pelletkerns der Filmüberzug nicht reißt. Hierbei überrascht insbesondere die Kombination hohe Flexibilität mit extrem niedriger Klebrigkeit, da normalerweise Polymere entweder hart, d. h. wenig flexibel und nicht klebrig oder weich, d. h. flexibel aber klebrig sind.

Die Polymerdispersionen sind niedrigviskos, so dass hohe Feststoffkonzentrationen in der Sprühsuspension und ein extrem kurzer Sprühprozess realisiert werden können. Die Feststoffkonzentrationen der Sprühsuspensionen betragen üblicherweise 10 bis 60 Gew.-%, bevorzugt 20 bis 50 Gew.-% und insbesondere 25 bis 40 Gew.-%. Die Konzentration bezogen auf festes erfindungsgemäß eingesetztes aminogruppenhaltiges Polymer liegen üblicherweise in einem Bereich von 5 bis 50 Gew.-%, bevorzugt von 10 bis 40 Gew.-% und insbesondere von 20 bis 35 Gew.-%. Die niedrigen Viskositäten sorgen für eine sehr feine und gleichmäßige Zerstäubung in der Sprühdüse, eine sehr gute Spreitung auf der Tabletten- oder Pelletoberfläche und eine schnelle und homogene Verfilmung. Die Einarbeitung von Farbstoffen und Pigmenten erfolgt auf üblichem Weg, ist aber extrem einfach und schnell und ist lösungsmittelfrei. Das einfache Handling resultiert aus der Tatsache, dass die erfindungsgemäß eingesetzten aminogruppenhaltigen Polymerdispersionen die Pigmente in der Sprühsuspension stabilisieren. Innerhalb von etwa 10 min lässt sich so eine sprühfertige Suspension herstellen.

Die Formulierungsgrundlage erfindungsgemäßer pharmazeutischer Mittel enthält bevorzugt pharmazeutisch akzeptable Hilfsstoffe. Pharmazeutisch akzeptabel sind die im Bereich der Pharmazie, der Lebensmitteltechnologie und angrenzenden Gebieten bekanntermaßen verwendbaren Hilfsstoffe, insbesondere die in einschlägigen Arzneibüchern (z. B. DAB, Ph. Eur., BP, USP, JP) gelisteten sowie andere Hilfsstoffe, deren Eigenschaften einer physiologischen Anwendung nicht entgegenstehen.

Geeignete Hilfsstoffe können sein: Gleitmittel, Netzmittel, emulgierende und suspendierende Mittel, konservierende Mittel, Antioxidantien, Antireizstoffe, Chelatbildner, Emulsionsstabilisatoren, Filmbildner, Gelbildner, Geruchsmaskierungsmittel, Harze, Hydrokolloide, Lösemittel, Lösungsvermittler, Neutralisierungsmittel, Permeationsbeschleuniger, Pigmente, Farbstoffe, Stabilisatoren, Sprengmittel, Trocknungsmittel, Trübungsmittel, Verdickungsmittel, Wachse, Weichmacher, Aromen, Süßstoffe, Hilfsstoffe zur Permeationserniedrigung etc. Eine diesbezügliche Ausgestaltung beruht auf fachmännischem Wissen, wie sie beispielsweise in Fiedler, H. P. Lexikon der Hilfsstoffe für Pharmazie, Kosmetik und angrenzende Gebiete, 4. Aufl., Aulendorf: ECV-Editio-Cantor-Verlag, 1996, dargestellt sind.

Besonders geeignete Weichmacher sind z. B.: Triethylcitrat, Tributylcitrat, Triacetin, Acetyltriethylcitrat, Labrasol, Glycofurol, Polypropylenglykol 400.

Die Permeabilität der Filmüberzüge kann durch Einarbeitung von anorganischen Feststoffen (Pigmenten wie z. B. Talkum, Kaolin, Titandioxid) oder lipophilen organischen Feststoffen wie Fetten Wachsen, Glyceride, Fettsäuren wie z. B. Stearinsäure, Fettatkohole wie z. B. Stearylalkohol weiter herabgesetzt werden.

Zur Herstellung der erfindungsgemäßen pharmazeutischen Mittel können die Wirkstoffe mit geeigneten Hilfsstoffen (Excipients) vermischt oder verdünnt werden. Hilfsstoffe können feste oder halbfeste Materialien sein, die als Vehikel, Träger oder Medium für den Wirkstoff dienen können. Die Zumischung weiterer Hilfsstoffe erfolgt gewünschtenfalls in der dem Fachmann bekannten Weise.

In gleicher Weise können auch veterinärmedizinische Darreichungsformen, insbesondere Rumen-stabile Formen, sowie Darreichungsformen mit Vitaminen, Carotinoiden, Spurenelementen, Nutraceuticals, Aminosäuren und Nahrungsergänzungsstoffen hergestellt werden. Letztere können auch als Lebensmittel oder Supplements gelten.

Ein weitere Gegenstand der Erfindung ist die Verwendung einer wässrigen Polymerdispersion Pd), wie zuvor definiert, zur Membranherstellung, in der Kosmetik, im Pflanzenschutz, für Saatgutcoating, in Lebensmitteln, Tierarzneimitteln, in Tiernahrung, als Klebrohstoffe, zu Papierherstellung, als Binde- oder Hilfsmittel für Leder und Textil, als mikrobizide Oberflächenbeschichtung, im Vliesstoffbereich, in Wasch- und Reinigungsmittel, zur Herstellung von Anstrichen, im Bausektor.

Ein weiterer Gegenstand der Erfindung ist die Verwendung einer wässrigen Polymerdispersion Pd), wie zuvor definiert als oder in Beschichtungsmittel(n) für die Kosmetik, Lebensmittel, Tiernahrung, die Textil-, Papier-, Druck-, Leder- und Klebstoffindustrie.

Die Erfindung wird anhand der folgenden, nicht einschränkenden Beispiele näher erläutert

Beispiel 1

Vorlage:

481,75 kg    vollentsalztes Wasser

(fortgesetzt)

| | |
|---|---|
| 5,59 kg | $C_{16}$-/$C_{18}$-Alkyl-Polyglykolether mit ca. 20 Ethylenoxideinheiten, Pharma Grade, 10%ige wässrige Lösung, |
| 4,58 kg | Natriumlaurylsulfat GMP, 15%ige wässrige Lösung |

Zugabe 1:

| | |
|---|---|
| 14,60 kg | vollentsalztes Wasser |
| 0,38 kg | Natriumpersulfat |

Zulauf 1:

| | |
|---|---|
| 248,52 kg | vollentsalztes Wasser |
| 86,43 kg | $C_{16}$-/$C_{18}$-Alkyl-Polyglykolether mit ca. 20 Ethylenoxideinheiten, Pharma Grade, 10%ige wässrige Lösung |
| 71,38 kg | Natriumlaurylsulfat GMP, 15%ige wässrige Lösung |

Zulauf 2:

| | |
|---|---|
| 172,00 kg | Diethylaminoethylmethacrylat |
| 258,00 kg | Methylmethacrylat |

Zulauf 3:

| | |
|---|---|
| 153,09 kg | vollentsalztes Wasser |
| 3,92 kg | Natriumpersulfat |

Durch geeignete technische Maßnahmen (Spülen mit Aceton und/oder Trockenblasen) wird sichergestellt, dass der Zulaufkessel (Zulauf 2) weitgehend wasserfrei ist. Die Zugabe 1 und der Zulauf 3 werden unmittelbar, d. h. 1 Stunde vor Beginn der Polymerisation, frisch angesetzt (Auflösen von festem Natriumpersulfat in vollentsalztem Wasser). Der dynamische Mischer (Megatron MT 3-61, Kinematica AG) wird vor Versuchsbeginn mit Wasser befüllt.

Der Polymerisationsreaktor (Kesselvolumen ca. 2050 l) und alle mit der Polymerdispersion in Kontakt kommenden Leitungen werden vor Versuchsbeginn mit einer 3%igen wässrigen Lösung Natronlauge gespült. Anschließend wird der Polymerisationsreaktor mit der Vorlage gefüllt. Die Vorlage wird vor Beginn der Reaktion evakuiert, einmal mit 5 bar Stickstoff begast, erneut evakuiert und mit Stickstoff auf Normaldruck gebracht. Anschließend wird die Vorlage unter Rühren auf 75 °C Reaktionstemperatur aufgeheizt. Bei Erreichen einer Innentemperatur von 70 °C wird die Zugabe 1 innerhalb von zwei Minuten zugegeben.

Zuläufe 1 und 2 werden dem Reaktor über den dynamischen Mischer (Drehzahleinstellung 5000 U/min) zudosiert, Zulauf 3 wird dem Reaktor über einen statischen Mischer zudosiert, der sich in der Leitungsstrecke zwischen dynamischem Mischer und Polymerisationsreaktor befindet.

Zulauf 1 wird unmittelbar vor den Zuläufen 2 und 3 gestartet. Die Zugabe von Zulauf 1 erfolgt im Verlauf von 1,75 Stunden, von Zulauf 2 innerhalb von 1,50 Stunden, von Zulauf 3 innerhalb von 3,75 Stunden.

Nach Beendigung von Zulauf 3 lässt man noch 2 Stunden unter Rühren bei 75 °C nachpolymerisieren. Anschließend wird der Reaktionsansatz auf Raumtemperatur gekühlt und Feststoffgehalt sowie pH-Wert bestimmt. Der pH-Wert während der Polymerisation (genauer: während der Zugabe der Monomeren) war bei Beispiel 1 und den folgenden Beispielen 2 bis 6 immer höher als 8,0. Die K-Werte wurden bei allen Beispielen 1 %ig in NMP bestimmt.

| Kenngrößen der Dispersion | Einheit | Messwert oder Beurteilung |
|---|---|---|
| Feststoffgehalt | (Gew.-%) | 32,7 |
| pH-Wert | | 9,0 |

Die Dispersion wird dann ultrafiltriert und die folgenden Kenngrößen bestimmt:

| Kenngrößen der Dispersion | Einheit | Messwert oder Beurteilung |
|---|---|---|
| Feststoffgehalt | (Gew.-%) | 30,5 |
| Viskosität | (mPas) | 6 |
| pH-Wert | | 9,2 |
| LD-Wert | (%) | 86 |
| K-Wert | | 50 |
| Mittlere Teilchengröße (bestimmt mittels AUZ) | (nm) | 100 |
| Methanol | (ppm) | 20 |
| Methacrylsäure | (ppm) | 60 |
| N,N-Diethylethanolamin | (ppm) | 280 |
| Lagerstabilität (18 Monate) | | exzellent, kein Bodensatz |
| AUZ = Analytische Ultrazentrifuge | | |

Beispiel 2

Man verfährt wie in Beispiel 1, aber setzt im Zulauf 3 nur 2,2 kg Natriumpersulfat ein, sowie im Zulauf 2 zusätzlich 0,4 kg Ethylhexylthioglykolat.

| Kenngrößen der Dispersion | Einheit | Messwert oder Beurteilung |
|---|---|---|
| Feststoffgehalt | (Gew.-%) | 31,9 |
| pH-Wert | | 9,1 |

Die Dispersion wird dann ultrafiltriert und die folgenden Kenngrößen bestimmt:

| Kenngrößen der Dispersion | Einheit | Messwert oder Beurteilung |
|---|---|---|
| Feststoffgehalt | (Gew.-%) | 30 |
| Viskosität | (mPas) | 6 |
| pH-Wert | | 9,2 |
| LD-Wert | (%) | 87 |
| K-Wert | | 52 |
| Mittlere Teilchengröße (bestimmt mittels AUZ) | (nm) | 105 |
| Methanol | (ppm) | 18 |
| Methacrylsäure | (ppm) | 48 |
| N,N-Diethylethanolamin | (ppm) | 240 |
| Lagerstabilität (nach 18 Monaten) | | exzellent, minimaler Bodensatz |

Beispiel 3

Man verfährt wie in Beispiel 1, aber setzt im Zulauf 2 193,5 kg Diethylaminoethylmethacrylat und 236,5 kg Methylmethacrylat ein.

| Kenngrößen der Dispersion | Einheit | Messwert oder Beurteilung |
|---|---|---|
| Feststoffgehalt | (Gew.-%) | 30,3 |

(fortgesetzt)

| Kenngrößen der Dispersion | Einheit | Messwert oder Beurteilung |
|---|---|---|
| pH-Wert | | 9,0 |

Die Dispersion wird dann ultrafiltriert und die folgenden Kenngrößen bestimmt:

| Kenngrößen der Dispersion | Einheit | Messwert oder Beurteilung |
|---|---|---|
| Feststoffgehalt | (Gew.-%) | 30 |
| Viskosität | (mPas) | 5 |
| pH-Wert | | 9,2 |
| LD-Wert | (%) | 89 |
| K-Wert | | 50 |
| Mittlere Teilchengröße (bestimmt mittels AUZ) | (nm) | 110 |
| Methanol | (ppm) | 22 |
| Methacrylsäure | (ppm) | 65 |
| N,N-Diethylethanolamin | (ppm) | 210 |
| Lagerstabilität (nach 18 Monaten) | | exzellent, minimaler Bodensatz |

Beispiel 4

Vorlage:

| 378,24 kg | vollentsalztes Wasser |
|---|---|
| 5,59 kg | $C_{16}$-/$C_{18}$-Alkyl-Polyglykolether mit ca. 20 Ethylenoxideinheiten, Pharma Grade, 10%ige wässrige Lösung, |
| 4,58 kg | Natriumlaurylsulfat GMP, 15%ige wässrige Lösung |

Zugabe 1:

| 14,60 kg | vollentsalztes Wasser |
|---|---|
| 0,38 kg | Natriumpersulfat |

Zulauf 1:

| 352,03 kg | vollentsalztes Wasser |
|---|---|
| 86,43 kg | $C_{16}$-/$C_{18}$-Alkyl-Polyglykolether mit ca. 20 Ethylenoxideinheiten, Pharma Grade, 10%ige wässrige Lösung |
| 71,38 kg | Natriumlaurylsulfat GMP, 15%ige wässrige Lösung |

Zulauf 2:

| 172,00 kg | Diethylaminoethylmethacrylat |
|---|---|
| 258,00 kg | Methylmethacrylat |

Zulauf 3:

| | |
|---|---|
| 153,09 kg | vollentsalztes Wasser |
| 3,92 kg | Natriumpersulfat |

Der Polymerisationsreaktor (Kesselvolumen ca. 2050 l) und alle mit der Polymerdispersion in Kontakt kommenden Leitungen werden vor Versuchsbeginn mit einer 3%igen wässrigen Lösung Natronlauge gespült. Anschließend wird der Polymerisationsreaktor mit der Vorlage gefüllt.

Die Vorlage wird vor Beginn der Reaktion evakuiert, einmal mit 5 bar Stickstoff begast, erneut evakuiert und mit Stickstoff auf Normaldruck gebracht. Anschließend wird die Vorlage unter Rühren auf 75 °C Reaktionstemperatur aufgeheizt. Bei Erreichen einer Innentemperatur von 70 °C wird die Zugabe 1 innerhalb von zwei Minuten zugegeben.

Zuläufe 1 und 2 werden dem Reaktor über den dynamischen Mischer (Drehzahleinstellung 5000 U/min) zudosiert, Zulauf 3 wird dem Reaktor über einen statischen Mischer zudosiert, der sich in der Leitungsstrecke zwischen dynamischem Mischer und Polymerisationsreaktor befindet.

Zulauf 1 wird unmittelbar vor den Zuläufen 2 und 3 gestartet. Die Zugabe von Zulauf 1 erfolgt im Verlauf von 1,75 Stunden, von Zulauf 2 innerhalb von 1,50 Stunden, von Zulauf 3 innerhalb von 3,75 Stunden.

Nach Beendigung von Zulauf 3 lässt man noch 2 Stunden unter Rühren bei 75 °C nachpolymerisieren. Anschließend wird der Reaktionsansatz auf Raumtemperatur gekühlt und Feststoffgehalt sowie pH-Wert bestimmt.

| Kenngrößen der Dispersion | Einheit | Messwert oder Beurteilung |
|---|---|---|
| Feststoffgehalt | (Gew.-%) | 31,7 |
| pH-Wert | | 9,0 |

Die Dispersion wird dann ultrafiltriert und die folgenden Kenngrößen bestimmt:

| Kenngrößen der Dispersion | Einheit | Messwert oder Beurteilung |
|---|---|---|
| Feststoffgehalt | (Gew.-%) | 30 |
| Viskosität | (mPas) | 6 |
| pH-Wert | | 9,2 |
| LD-Wert | (%) | 85 |
| K-Wert | | 50,5 |
| Mittlere Teilchengröße (bestimmt mittels AUZ) | (nm) | 105 |
| Methanol | (ppm) | 20 |
| Methacrylsäure | (ppm) | 40 |
| N,N-Diethylethanolamin | (ppm) | 210 |
| Lagerstabilität (18 Monate) | | exzellent, kein Bodensatz |

Beispiel 5

Man verfährt wie in Beispiel 4, aber setzt in der Zugabe 1 und im Zulauf 3 anstelle Natriumpersulfat Kaliumpersulfat ein.

| Kenngrößen der Dispersion | Einheit | Messwert oder Beurteilung |
|---|---|---|
| Feststoffgehalt | (Gew.-%) | 30,8 |
| pH-Wert | | 9,1 |

Die Dispersion wird dann ultrafiltriert und die folgenden Kenngrößen bestimmt:

| Kenngrößen der Dispersion | Einheit | Messwert oder Beurteilung |
|---|---|---|
| Feststoffgehalt | (Gew.-%) | 30 |

(fortgesetzt)

| Kenngrößen der Dispersion | Einheit | Messwert oder Beurteilung |
|---|---|---|
| Viskosität | (mPas) | 6 |
| pH-Wert | | 9,2 |
| LD-Wert | (%) | 89 |
| K-Wert | | 51 |
| Mittlere Teilchengröße (bestimmt mittels AUZ) | (nm) | 110 |
| Methanol | (ppm) | 15 |
| Methacrylsäure | (ppm) | 55 |
| N,N-Diethylethanolamin | (ppm) | 190 |
| Lagerstabilität (nach 18 Monaten) | | exzellent, minimaler Bodensatz |

Beispiel 6

Man verfährt wie in Beispiel 4, aber setzt in der Zugabe 1 und im Zulauf 3 anstelle Natriumpersulfat Ammoniumpersulfat ein und stellt den pH-Wert jeweils mit wässriger NaOH auf pH 9 ein.

| Kenngrößen der Dispersion | Einheit | Messwert oder Beurteilung |
|---|---|---|
| Feststoffgehalt | (Gew.-%) | 30,7 |
| pH-Wert | | 8,9 |

Die Dispersion wird dann ultrafiltriert und die folgenden Kenngrößen bestimmt:

| Kenngrößen der Dispersion | Einheit | Messwert oder Beurteilung |
|---|---|---|
| Feststoffgehalt | (Gew.-%) | 30 |
| Viskosität | (mPas) | 7 |
| pH-Wert | | 9,1 |
| LD-Wert | (%) | 85 |
| K-Wert | | 51 |
| Mittlere Teilchengröße (bestimmt mittels AUZ) | (nm) | 115 |
| Methanol | (ppm) | 25 |
| Methacrylsäure | (ppm) | 50 |
| N,N-Diethylethanolamin | (ppm) | 210 |
| Lagerstabilität (nach 18 Monaten) | | exzellent, minimaler Bodensatz |

In einer Variante der zuvor angegebenen Beispiele lassen sich selbstverständlich auch Dispersionen mit einem von 30 Gew.-% verschiedenen Feststoffgehalt herstellen. Hierzu kann beispielsweise vollentsalztes Wasser

a) zu der Vorlage und/oder
b) zum Zulauf 1 und/oder
c) zum Zulauf 3

hinzugefügt (mit dem Ziel eines geringeren Feststoffgehalts) oder entfernt werden (mit dem Ziel eines höheren Feststoffgehalts).

In einer anderen Ausführungsform kann beispielsweise Wasser aus der Vorlage in den Zulauf 1 und/oder den Zulauf 3 gegeben werden, wobei sich dann der Feststoffgehalt nicht ändert. Die Umverteilung kann aber auch in der Weise erfolgen, dass das aus der Vorlage und/oder dem Zulauf 1 und/oder dem Zulauf 3 entnommene Wasser ganz oder teilweise in einen neuen Zulauf ("Zulauf 4") gegeben wird, wobei der Zulauf 4 dann parallel zur Polymerisation, zeitverschoben zur Polymerisation oder nach der Polymerisation kontinuierlich oder auf einmal zugegeben werden kann. Dies kann beispielsweise zur Anpassung der Rezeptur an die vorhandenen Kesselgrößen dienen, z. B. eine Überfüllung bzw. eine Nachbefüllung des Zulaufs 1 zu vermeiden.

Selbstverständlich kann es von Vorteil sein, die in den Beispielen offenbarte Emulgatorverteilung auf die Vorlage und den Zulauf 1 dahingehend zu variieren, dass anionischer und/oder nichtionischer Emulgator von der Vorlage in den Zulauf 1 (oder umgekehrt) gegeben wird. Selbstverständlich ist es auch möglich, dass anionischer und/oder nichtionischer Emulgator aus der Vorlage und/oder Zulauf 1 in einen zusätzlichen Zulauf 4 (vgl. oben) gegeben werden. Bei all diesen Maßnahmen bleibt die Gesamtmenge an Emulgator vorzugsweise konstant.

Selbstverständlich kann es auch von Vorteil sein, wenn der Zuläufe 1 und/oder der Zulauf 2 und/oder der Zulauf 3 nicht mit einer konstanten Rate zudosiert werden, sondern mit nicht konstanter Rate zugefahren werden. Beispielsweise kann der Initiatorzulauf während der Polymerisationsphase (d. h. während der Zugabe von Zulauf 2) mit einer höheren Rate dosiert werden als nach Beendigung des Zulaufs 2.

Beispiel zur Filmherstellung

Eine 30%ige Dispersion aus Diethylaminoethylmethacrylat - Methylmethacrylat Copolymer aus dem Beispiel 1 wurde unter Rühren mit 15 % Triethylcitrat, bezogen auf den Feststoff, versetzt und auf einem Filmziehgerät (Erichsen Coatmaster) ausgerakelt und bei 45 °C Plattentemperatur zu einem Film getrocknet. Die Filmdicke betrug 100$\mu$m.

Folgende Eigenschaften wurden ermittelt:

| | |
|---|---|
| Reißdehnung (elongation at break): | 93 % |
| Zugfestigkeit (tensile strength): | 9,8 N/mm2 |
| Wasserdampfpermeabilität nach DIN 53122 bei 93 % r. F.: | 58 g/(m2/d)/100$\mu$m Film |
| Auflösungszeit in 0,1 N-HCl | 2 min 50 s |
| Auflösungszeit in Phosphatpuffer pH 6,8 | > 120 min |
| Klebrigkeit nach Hoessel bei 20 °C/80 % r.F. | 0,25 (mit Triacetin anstelle von Triethylcitrat als Weichmacher) |
| 30 °C/75 % r.F. | 0,25 (mit Triacetin anstelle von Triethylcitrat als Weichmacher) |

Methode beschrieben in Cosmetics and Toiletries, 111 (8), 73ff (1996); Skala von 0 (nicht klebrig) bis 5 (klebrig), r. F. = relative Feuchte

Beispiele zum Coating von Darreichungsformen

Coatingbeispiel 1: Propranolol-HCl 40 mg Filmtabletten

Zusammensetzung der Tabletten

| Substanz | Zusammensetzung pro Tablette [mg] |
|---|---|
| Propranolol-HCl | 40 |
| Ludipress | 97,5 |
| Avicel PH 105 | 97,5 |
| Kollidon VA 64 | 12,5 |
| Magnesiumstearat | 2,5 |
| Summe | 250 |

Format: 9 mm, Drageeform

Zusammensetzung der Sprührezeptur

| Substanz | Anteil im Film [%] | Anteil in der Suspension [%] |
|---|---|---|
| 30%ige wässrige Dispersion aus dem Beispiel 2 | 67,83 | 45,22 |

(fortgesetzt)

| Substanz | Anteil im Film [%] | Anteil in der Suspension [%] |
|---|---|---|
| Triethylcitrat | 10,17 | 2,03 |
| Eisenoxid rot | 2 | 0,4 |
| Talkum | 20 | 4 |
| Wasser, entmineralisiert | - | 48,35 |
| Summe | 100 | 100 |

Der Weichmacher Triethylcitrat wurde zu der Polymerdispersion gegeben und weiter rühren gelassen. Talkum und Eisenoxid rot wurden in Wasser angeschlemmt und mittels eines High-Shear-Mixers homogenisiert. Anschließend wurden beide Phasen gemischt, indem die Pigmentsuspension zu der Polymerdispersion gegeben wurde.

Coatingparameter:

Gecoatet wurde in einem Horizontaltrommelcoater "Accela Cota 24" der Fa. Manesty.

Folgende Bedingungen wurden eingestellt bzw. ergaben sich aus den Einstellungen:

| | |
|---|---|
| Sprühdüse | Schlick 937 mit 1mm Flüssigkeitseinsatz |
| Anzahl der Sprühdüsen | 1 |
| Befüllung | 10 kg Propranolol-HCl Kerne |
| Abstand Kernbett zu Düse | 20 cm |
| Sprühdruck | 1,5 bar |
| Formierluftdruck | 0,5 bar |
| Zulufttemperatur | 60 C |
| Ablufttemperatur | 36 °C |
| Kesseldrehzahl | 15 UPM |
| Sprührate | 40 g/min. |
| Sprühzeit | 55 min. |
| Trocknung | ca. 5 min. |
| Auftragsmenge | 6 mg/cm$^2$ |

Eigenschaften der Filmtabletten

Freisetzung

Paddle, 50 UPM, 37 °C, 1000 ml

| | 0,08 N HCl | Phosphatpuffer pH 6,8 |
|---|---|---|
| nach 45 min. | >90 % | <5 % |

Zerfallszeit

Zerfalltester Erweka Typ ZT 74, 37 °C, 800 ml

| 0,08 N HCl (min:s) | Phosphatpuffer pH 6,8 (min:s) |
|---|---|
| 4:30 | > 45 min |

Coatingbeispiel 2: Enalapril 10 mg Filmtabletten

Zusammensetzung der Tabletten

| Substanz | Zusammensetzung pro Tablette [mg] |
|---|---|
| Enalapril | 10 |
| Avicel PH 102 | 120 |
| Di-Tab | 60 |
| Kollidon CL | 8 |
| Magnesiumstearat | 2 |
| Summe | 200 |

Format: 7 mm, Drageeform

Zusammensetzung der Sprührezeptur

| Substanz | Anteil im Film [%] | Anteil in der Suspension [%] |
|---|---|---|
| 30%ige wässrige Dispersion aus dem Beispiel 3 | 62,4 | 41,60 |
| Tributylcitrat | 9,36 | 1,87 |
| | | |
| Polyvinylalkohol 5-88 | 6,24 | 1,25 |
| Wasser, entmineralisiert | - | 30,88 |
| | | |
| Titandioxid | 2,00 | 0,40 |
| Talkum | 20,00 | 4,00 |
| Wasser, entmineralisiert | - | 20,00 |
| Summe | 100,00 | 100,00 |

Der Weichmacher Tributylcitrat wurde zu der Polymerdispersion gegeben und weiter rühren gelassen. Talkum und Titandioxid wurden in Wasser angeschlemmt und mittels eines High-Shear-Mixers homogenisiert. Polyvinylalkohol wird in Wasser auf 85 °C erhitzt und gelöst. Die erkaltete Lösung wird in die Polymerdispersion eingerührt. Anschließend wurde die Pigmentsuspension unter Rühren zugegeben.

Coatingparameter

Gecoatet wurde in einem Horizontaltrommelcoater "Accela Cota 24" der Fa. Manesty.

Folgende Bedingungen wurden eingestellt bzw. ergaben sich aus den Einstellungen:

| Sprühdüse | Schlick 937 mit 1 mm Flüssigkeitseinsatz |
|---|---|
| Anzahl der Sprühdüsen | 1 |
| Befüllung | 12 kg Enalaprilkerne |
| Abstand Kernbett zu Düse | 20 cm |
| Sprühdruck | 1,5 bar |
| Formierluftdruck | 0,5 bar |
| Zulufttemperatur | 65 °C |
| Ablufttemperatur | 38 °C |
| Kesseldrehzahl | 15 UPM |
| Sprührate | 45 g/min. |
| Sprühzeit | 50 min. |
| Trocknung | ca. 5 min. |
| Auftragsmenge | 5 mg/cm$^2$ |

Ergebnisse

Freisetzung: Paddle, 50 UPM, 37 °C, 1000 ml

|  | 0,08 N HCl | Phosphatpuffer pH 6,8 |
|---|---|---|
| nach 45 min. | >90 % | <10 % |

Zerfallszeit: Zerfalltester Erweka Typ ZT 74, 37 °C

| 0,08 N HCl (min:s) | Phosphatpuffer pH 6,8 (min:s) |
|---|---|
| 3:59 | >45 |

Stabilität: Lagerung bei 30 °C/70 % r. F. über 1 Jahr

|  | Gehalt Enalapril (%) 0 Monate | Gehalt Enalapril (%) 12 Monate |
|---|---|---|
| Kern | 100,2 | 92,3 |
| Filmtablette | 100,7 | 100,1 |

Coatingbeispiel 3: gecoatete Ibuprofen-Minipellets

Zusammensetzung der Pellets

| Substanz | Zusammensetzung pro Pellet [%] |
|---|---|
| Ibuprofen | 100 |

28

Pelletgröße 200 bis 400 μm

Zusammensetzung der Sprührezeptur

| Substanz | Anteil im Film [%] | Anteil in der Suspension [%] |
|---|---|---|
| 30%ige wässrige Dispersion aus dem Beispiel 4 | 65,42 | 43,61 |
| Triacetin | 13,08 | 2,62 |
| Talkum | 21,5 | 4,3 |
| Wasser, entmineralisiert | - | 49,47 |
| Summe | 100 | 100 |

Der Weichmacher Triacetin wurde zu der Polymerdispersion gegeben und rühren gelassen. Talkum wurde in Wasser angeschlemmt und mittels eines High-Shear-Mixers homogenisiert. Anschließend wurden beide Zubereitungen gemischt.

Coatingparameter

Gecoatet wurde in einem Wirbelschichtgranulator "Glatt GPCG 3.1" der Fa. Glatt.

| Sprühdüse | 1 mm Durchmesser |
|---|---|
| Anzahl der Sprühdüsen | 1 |
| Befüllung | 2,5 kg Ibuprofenpellets 200-400μm |
| Verfahren | Bottom-Spray (Wurster) |
| Sprühdruck | 1,0 bar |
| Zulufttemperatur | 60 °C |
| Ablufttemperatur | 46 °C |
| Sprührate | 12 g/min. |
| Sprühzeit | 4 h |
| Trocknung | ca. 5 min. |
| Auftragsmenge/Gewichtszuwachs | 20 % |

Tablettierung zu MUPS-Tabletten

Durch Mischen und Verpressen der oben hergestellten gecoateten Pellets und mikrokristalline Cellulose Typ 102 ist es möglich, eine Tablette herzustellen, die beim Zerfall wieder in die Pellets zerfällt.

| Substanz | Zusammensetzung pro Tablette [mg] |
|---|---|
| gecoatete Ibuprofen Minipellets | 120 |
| Mikrokristalline Cellulose Typ 102 | 258 |
| Kollidon CL | 20 |
| Magnesiumstearat | 2 |
| Summe | 400 |
| Format: 11 mm, flach, facettiert | |

Tabletten- bzw. Pelleteigenschaften:

Freisetzung, Pellets, Paddle, 50 UPM, 37 °C, 1000 ml, Einwaage 250 mg

|  | Acetatpuffer pH 4,5 | Acetatpuffer pH 6,8 |
|---|---|---|
| nach 45 min. | >90 % | <10 % |

Tabletten:

Paddle, 50 UPM, 37 °C, 1000 ml

|  | Acetatpuffer pH 4,5 | Acetatpuffer pH 6,8 |
|---|---|---|
| nach 45 min. | >90 % | <15 % |

Zerfall Tablette:

Zerfalltester Erweka Typ ZT 74, 37 °C

| Acetatpuffer pH 4,5 | Acetatpuffer pH 6,8 |
|---|---|
| 2:29 | 2:45 |

Coatingbeispiel 4: gecoatete Coffein-Pellets

Zusammensetzung der Pellets

| Substanz | Zusammensetzung pro Pellet [%] | | |
|---|---|---|---|
| Coffein, feines Pulver | 20 | | |
| Avicel PH 101 (MCC) | 38,75 | 20 | Herstellung durch |
| Granulac 230 (Lactose) | 38,75 | | Extrusion, Pelletgröße |
| Kollidon VA 64 | 2,5 | | 0,7 - 1,4 mm |

Zusammensetzung der Sprührezeptur

| Substanz | Anteil im Film [%] | Anteil in der Suspension [%] |
|---|---|---|
| 30%ige wässrige Dispersion aus dem Beispiel 5 | 62,61 | 41,74 |
| Acetyltriethylcitrat | 9,39 | 1,88 |
| Eisenoxid gelb | 3 | 0,6 |
| Kaolin | 25 | 5 |
| Wasser, entmineralisiert | - | 50,78 |

(fortgesetzt)

| Substanz | Anteil im Film [%] | Anteil in der Suspension [%] |
|---|---|---|
| Summe | 100 | 100 |

Der Weichmacher Acetyltriethylcitrat wurde direkt zu der kationischen Polymerdispersion zugegeben und rühren gelassen. Talkum und Eisenoxid gelb wurden in Wasser angeschlemmt und mittels eines Ultraturrax homogenisiert. Anschließend wurden beide Phasen gemischt indem die Pigmentsuspension zu der Polymerdispersion gegeben wurde.

Coatingparameter:

Gecoatet wurde in einem Wirbelschichtgranulator "Glatt GPCG 3.1" der Fa. Glatt.

Folgende Bedingungen wurden eingestellt bzw. ergaben sich aus den Einstellungen:

| | |
|---|---|
| Sprühdüse | 1 mm Durchmesser |
| Anzahl der Sprühdüsen | 1 |
| Befüllung | 2,5 kg Coffein-Pellets 0,7-1,4mm |
| Verfahren | Top-Spray |
| Sprühdruck | 1,0 bar |
| Zulufttemperatur | 60 °C |
| Ablufttemperatur | 43 °C |
| Sprührate | 14 g/min. |
| Sprühzeit | 135 min. |
| Trocknung | ca. 5 min. |
| Auftragsmenge/Gewichtszuwachs | 15 % |

Freisetzung:

Paddle, 50 UPM, 37 °C, 1000 ml, Einwaage 300 mg

| | 0,08 N HCl | Phosphatpuffer pH 6,8 |
|---|---|---|
| nach 45 min. | >95 % | <10 % |

Coatingbeispiel 5: gecoatete Chininsulfat-Minipellets

Zusammensetzung der Pellets

| Substanz | Zusammensetzung pro Pellet [%] | |
|---|---|---|
| Chininsulfat * 2 H$_2$O | 10 | Pelletgröße 125 - 300 μm |
| Avicel PH 101 (MCC) | 46,75 | |
| Granulac 230 (Lactose) | 40,75 | |
| Kollidon VA 64 | 2,5 | |

Zusammensetzung der Sprührezeptur

| Substanz | Anteil im Film [%] | Anteil in der Suspension [%] |
|---|---|---|
| 30%ige wässrige Dispersion aus dem Beispiel 6 | 68,26 | 45,51 |
| Triacetin | 10,24 | 2,05 |
| Indigotinlack | 1,5 | 0,3 |
| Talkum | 20 | 4 |
| Wasser, entmineralisiert | - | 48,14 |
| Summe | 100 | 100 |

Der Weichmacher Triacetin wurde direkt zu der Polymerdispersion zugegeben und rühren gelassen. Talkum und Indigotinlack wurden in Wasser angeschlemmt und mittels eines Ultraturrax homogenisiert. Anschließend wurden beide Zubereitungen gemischt, indem die Pigmentsuspension zu der Polymerdispersion gegeben wurde.

Coatingparameter:

Gecoatet wurde in einem Wirbelschichtgranulator "Glatt GPCG 3.1" der Fa. Glatt.
Folgende Bedingungen wurden eingestellt bzw. ergaben sich aus den Einstellungen:

| | |
|---|---|
| Sprühdüse | 1 mm Durchmesser |
| Anzahl der Sprühdüsen | 1 |
| Befüllung | 1,5 kg Chininsulfatpellets 125-300 $\mu$m |
| Verfahren | Bottom-Spray (Wurster) |
| Sprühdruck | 1,0 bar |
| Zulufttemperatur | 60 °C |
| Ablufttemperatur | 47 °C |
| Sprührate | 10 g/min. |
| Sprühzeit | 225 min. |
| Trocknung | ca. 5 min. |
| Auftragsmenge/Gewichtszuwachs | 30 % |

Freisetzung:

Paddle, 50 UPM, 37 °C, 1000 ml, Einwaage 1000 mg

| | 0,08 N HCl | Phosphatpuffer pH 6,8 |
|---|---|---|
| nach 45 min. | >90 % | <10 % |

**Patentansprüche**

1. Verfahren zur Herstellung einer wässrigen Polymerdispersion Pd), durch radikalische Emulsionspolymerisation eines Monomergemischs M), enthaltend

a) N,N-Diethylaminoethylmethacrylat, und

b) wenigstens eine radikalisch polymerisierbare Verbindung, ausgewählt unter Estern $\alpha,\beta$-ethylenisch unge-sättigter Mono- und Dicarbonsäuren mit $C_1$-$C_8$-Alkanolen,

in einem wässrigen Medium bei einem pH-Wert von wenigstens 8.

2. Verfahren nach Anspruch 1, wobei als Komponente b) Methylmethacrylat eingesetzt wird.

3. Verfahren nach einem der vorhergehenden Ansprüche, bei dem ein Monomergemisch M) eingesetzt wird, das

- 25 bis 65 Gew.- %, bezogen auf das Gesamtgewicht der zur Polymerisation eingesetzten Monomere, N,N-Diethylaminoethylmethacrylat a), und
- 35 bis 75 Gew.- %, bezogen auf das Gesamtgewicht der zur Polymerisation eingesetzten Monomere, wenigstens einer Verbindung b)

enthält.

4. Verfahren nach einem der vorhergehenden Ansprüche, bei dem ein Monomergemisch M) eingesetzt wird, das aus

- 43 bis 47 Gew.- %, bezogen auf das Gesamtgewicht der zur Polymerisation eingesetzten Monomere, N,N-Diethylaminoethylmethacrylat a), und
- 53 bis 57 Gew.- %, bezogen auf das Gesamtgewicht der zur Polymerisation eingesetzten Monomere, wenigstens einer Verbindung b)

besteht.

5. Verfahren nach einem der vorhergehenden Ansprüche, wobei zur radikalischen Emulsionspolymerisation wenigstens ein anorganisches Peroxid als Initiator eingesetzt wird, wobei der Initiator ausgewählt ist unter Ammonium- und Alkaliperoxodisulfaten.

6. Verfahren nach Anspruch 5, wobei zur radikalischen Emulsionspolymerisation eine wässrige Natriumperoxodisulfat-Lösung oder Kaliumperoxodisulfat-Lösung als Initiator eingesetzt wird, die unmittelbar vor ihrem Einsatz zur Polymerisation hergestellt wird oder deren pH-Wert vor ihrem Einsatz zur Polymerisation durch Zugabe wenigstens einer Base auf einen Wert von größer als 2, , eingestellt wird.

7. Verfahren nach einem der vorhergehenden Ansprüche, wobei vor Beginn der radikalischen Emulsionspolymerisation kein Monomer in der Polymerisationszone vorgelegt wird.

8. Verfahren nach einem der vorhergehenden Ansprüche, wobei die radikalische Emulsionspolymerisation nicht in Gegenwart eines Saatlatex erfolgt.

9. Verfahren nach einem der vorhergehenden Ansprüche, wobei das N,N-Diethylaminoethylmethacrylat a) unmittelbar vor seinem Einsatz zur Polymerisation in einer Mischvorrichtung mit Wasser, wenigstens einem Emulgator und gegebenenfalls wenigstens einem weiteren Monomer unter Erhalt einer Monomerenemulsion vermischt wird.

10. Verfahren nach einem der vorhergehenden Ansprüche, wobei vor Beginn der radikalischen Emulsionspolymerisation alle mit der wässrigen Polymerdispersion Pd) in Kontakt kommende Anlagenteile mit einer Base in Kontakt gebracht werden.

11. Wässrige Polymerdispersion Pd), erhältlich durch ein Verfahren, wie in einem der Ansprüche 1 bis 10 definiert.

12. Wässrige, durch Verringerung des pH-Werts lösliche Polymerdispersion Pd), enthaltend

- wenigstens ein Polymer, das

a) N,N-Diethylaminoethylmethacrylat, und
b) wenigstens eine radikalisch polymerisierbare Verbindung, ausgewählt unter Estern $\alpha,\beta$-ethylenisch un-gesättigter Mono- und Dicarbonsäuren mit $C_1$-$C_8$-Alkanolen,

einpolymerisiert enthält,
- wenigstens einen Emulgator, ausgewählt unter anionischen und nichtionischen Emulgatoren, und
- Wasser.

**13.** Polymerdispersion nach Anspruch 11, enthaltend ein Polymer, das

- 43 bis 47 Gew.- %, bezogen auf das Gesamtgewicht der zur Polymerisation eingesetzten Monomere, N,N-Diethylaminoethylmethacrylat a), und
- 53 bis 57 Gew.- %, bezogen auf das Gesamtgewicht der zur Polymerisation eingesetzten Monomere, wenigstens einer Verbindung b)

als einzige Monomere einpolymerisiert enthält.

**14.** Überzugsmittel, enthaltend eine wässrige Polymerdispersion Pd), wie in einem der Ansprüche 11 bis 13 definiert, oder eine daraus durch Trocknung erhältliche Polymerzusammensetzung.

**15.** Pharmazeutisches Mittel oder Lebensmittel enthaltend

A) eine Polymerzusammensetzung, erhältlich durch Trocknen und/oder Verfilmen einer Polymerdispersion, wie in einem der Ansprüche 11 bis 13 definiert,
B) wenigstens einen pharmazeutisch akzeptablen Wirkstoff oder einen Nahrungsergänzungsstoff, und
C) gegebenenfalls wenigstens einen pharmazeutisch oder im Lebensmittelbereich akzeptablen Hilfsstoff.

**16.** Pharmazeutisches Mittel oder Lebensmittel nach Anspruch 15 in Form einer oralen Darreichungsform, umfassend einen Überzug auf Basis einer wässrigen Polymerdispersion Pd), wie in einem der Ansprüche 1 bis 10 definiert oder einer daraus durch Trocknung erhältlichen Polymerzusammensetzung.

**17.** Verwendung einer wässrigen Polymerdispersion Pd), wie in einem der Ansprüche 11 bis 13 definiert zur Membranherstellung, in der Kosmetik, im Pflanzenschutz, für Saatgutcoating, in Lebensmitteln, in Tierarzneimitteln, in Tiernahrung, als Klebrohstoffe, zur Papierherstellung, als Binde- oder Hilfsmittel für Leder und Textil, als mikrobizide Oberflächenbeschichtung, im Vliesstoffbereich, in Wasch- und Reinigungsmittel, zur Herstellung von Anstrichen, im Bausektor.

**18.** Verwendung einer wässrigen Polymerdispersion Pd), wie in einem der Ansprüche 11 bis 13 definiert als oder in Beschichtungsmittel(n) für Kosmetik, Lebensmittel, Tierarzneimittel, Tiernahrung, Saatgut, die Textil-, Papier-, Druck-, Leder- und Klebstoffindustrie.

**Claims**

**1.** A process for preparing an aqueous polymer dispersion Pd) by free-radical emulsion polymerization of a monomer mixture M), comprising

a) N,N-diethylaminoethyl methacrylate, and
b) at least one compound capable of free-radical polymerization selected from esters of $\square,\square$-ethylenically unsaturated mono- and dicarboxylic acids with $C_1$-$C_8$ alkanols,

in an aqueous medium at a pH of at least 8.

**2.** The process according to claim 1, where methyl methacrylate is employed as component b).

**3.** The process as claimed in either of the preceding claims, in which a monomer mixture M) which comprises

- 25 to 65% by weight, based on the total weight of the monomers employed for the polymerization, of N,N-diethylaminoethyl methacrylate a), and
- 35 to 75% by weight, based on the total weight of the monomers employed for the polymerization, of at least one compound b),
is employed.

**4.** The process according to any of the preceding claims, in which a monomer mixture M) which consists of

- 43 to 47% by weight, based on the total weight of the monomers employed for the polymerization, of N,N-diethylaminoethyl methacrylate a), and
- 53 to 57% by weight, based on the total weight of the monomers employed for the polymerization, of at least one compound b),

is employed.

**5.** The process according to any of the preceding claims, where at least one inorganic peroxide is employed as initiator for the free-radical emulsion polymerization, where the initiator is selected from ammonium and alkali metal peroxodisulfates.

**6.** The process according to claim 5, where an aqueous sodium peroxodisulfate solution or potassium peroxodisulfate solution which is prepared directly before use thereof for the polymerization, or whose pH is adjusted to a value greater than 2, by addition of at least one base before use thereof for the polymerization, is employed as initiator for the free-radical emulsion polymerization.

**7.** The process according to any of the preceding claims, where no monomer is introduced into the polymerization zone before starting the free-radical emulsion polymerization.

**8.** The process according to any of the preceding claims, where the free-radical emulsion polymerization does not take place in the presence of a seed latex.

**9.** The process according to any of the preceding claims, where the N,N-diethylaminoethyl methacrylate a) is mixed in a mixing device directly before use thereof for the polymerization with water, at least one emulsifier and optionally at least one further monomer to obtain a monomer emulsion.

**10.** The process according to any of the preceding claims, where all the parts of the system which come into contact with the aqueous polymer dispersion Pd) are brought into contact with a base before starting the free-radical emulsion polymerization.

**11.** An aqueous polymer dispersion Pd) obtainable by a process as defined in any of claims 1 to 10.

**12.** An aqueous polymer dispersion Pd) which is soluble through reduction in the pH and comprises

- at least one polymer which comprises

a) N,N-diethylaminoethyl methacrylate, and
b) at least one compound capable of free radical polymerization and selected from esters of $\square,\square$-ethylenically unsaturated mono- and dicarboxylic acids with $C_1$-$C_8$-alkanols,

as copolymerized units,
- at least one emulsifier selected from anionic and nonionic emulsifiers, and
- water.

**13.** The polymer dispersion according to claim 11, comprising a polymer which comprises

- 43 to 47% by weight, based on the total weight of the monomers employed for the polymerization, of N,N-diethylaminoethyl methacrylate a), and
- 53 to 57% by weight, based on the total weight of the monomers employed for the polymerization, of at least one compound b)

as only monomers in the copolymer.

**14.** A coating composition comprising an aqueous polymer dispersion Pd) as defined in any of claims 11 to 13, or a polymer composition obtainable therefrom by drying.

**15.** A pharmaceutical composition or food product comprising

A) a polymer composition obtainable by drying and/or forming a film of a polymer dispersion as defined in any of claims 11 to 13,
B) at least one pharmaceutically acceptable active ingredient or a dietary supplement, and
C) optionally at least one pharmaceutically acceptable excipient or auxiliary acceptable in the food products sector.

**16.** The pharmaceutical composition or food product according to claim 15 in the form of an oral dosage form comprising a coating based on an aqueous polymer dispersion Pd) as defined in any of claims 1 to 10, or a polymer composition obtainable therefrom by drying.

**17.** The use of an aqueous polymer dispersion Pd) as defined in any of claims 11 to 13 for producing membranes, in cosmetics, in crop protection, for seed coating, in food products, in veterinary medicaments, in animal nutrition, as adhesive raw material, for paper manufacture, as binder or auxiliary for leather and textile, as microbicidal surface coating, in the nonwoven sector, in detergents and cleaners, for producing paints, in the building sector.

**18.** The use of an aqueous polymer dispersion Pd) as defined in any of claims 11 to 13 as or in coating composition(s) for cosmetics, food products, veterinary medicaments, animal nutrition, seeds, and the textile, paper, printing, leather and adhesives industries.

## Revendications

**1.** Procédé pour la préparation d'une dispersion aqueuse de polymère Pd), par polymérisation radicalaire en émulsion d'un mélange de monomères M), contenant

a) du méthacrylate de N,N-diéthylaminoéthyle, et
b) au moins un composé polymérisable par voie radicalaire, choisi parmi les esters d'acides monocarboxyliques et d'acides dicarboxyliques éthyléniquement $\alpha,\beta$-insaturés avec des $C_1$-$C_8$-alcanols,

dans un milieu aqueux à un pH d'au moins 8.

**2.** Procédé selon la revendication 1, du méthacrylate de méthyle étant utilisé comme composant b).

**3.** Procédé selon l'une quelconque des revendications précédentes, dans lequel est utilisé un mélange de monomères M) qui contient

- 25 à 65% en poids, par rapport au poids total des monomères utilisés pour la polymérisation, de méthacrylate de N,N-diéthylaminoéthyle a), et
- 35 à 75% en poids, par rapport au poids total des monomères utilisés pour la polymérisation, d'au moins un composé b).

**4.** Procédé selon l'une quelconque des revendications précédentes, dans lequel est utilisé un mélange de monomères M) qui est constitué par

- 43 à 47% en poids, par rapport au poids total des monomères utilisés pour la polymérisation, de méthacrylate de N,N-diéthylaminoéthyle a), et
- 53 à 57% en poids, par rapport au poids total des monomères utilisés pour la polymérisation, d'au moins un composé b).

**5.** Procédé selon l'une quelconque des revendications précédentes, au moins un peroxyde inorganique étant utilisé comme initiateur pour la polymérisation radicalaire en émulsion, l'initiateur étant choisi parmi les peroxodisulfates d'ammonium et de métal alcalin.

**6.** Procédé selon la revendication 5, une solution aqueuse de peroxodisulfate de sodium ou de peroxodisulfate de potassium étant utilisée comme initiateur pour la polymérisation radicalaire en émulsion, qui est préparée juste avant son utilisation pour la polymérisation ou dont le pH est réglé avant son utilisation pour la polymérisation par addition d'au moins une base à une valeur supérieure à 2.

7. Procédé selon l'une quelconque des revendications précédentes, aucun monomère n'étant disposé au préalable dans la zone de polymérisation avant le début de la polymérisation radicalaire en émulsion.

8. Procédé selon l'une quelconque des revendications précédentes, la polymérisation radicalaire en émulsion n'ayant pas lieu en présence d'un latex semence.

9. Procédé selon l'une quelconque des revendications précédentes, le méthacrylate de N,N-diéthylaminoéthyle a) étant mélangé juste avant son utilisation pour la polymérisation, dans un dispositif de mélange, avec de l'eau, au moins un émulsifiant et le cas échéant au moins un autre monomère avec obtention d'une émulsion de monomères.

10. Procédé selon l'une quelconque des revendications, toutes les parties d'installation entrant en contact avec la dispersion aqueuse de polymère Pd) étant mises en contact avec une base avant le début de la polymérisation radicalaire en émulsion.

11. Dispersion aqueuse de polymère Pd), pouvant être obtenue par un procédé tel que défini dans l'une quelconque des revendications 1 à 10.

12. Dispersion aqueuse de polymère Pd), soluble par diminution du pH, contenant

- au moins un polymère qui contient, sous forme copolymérisée

a) du méthacrylate de N,N-diéthylaminoéthyle, et
b) au moins un composé polymérisable par voie radicalaire, choisi parmi les esters d'acides monocarboxyliques et d'acides dicarboxyliques éthyléniquement $\alpha,\beta$-insaturés avec des $C_1$-$C_8$-alcanols,

- au moins un émulsifiant, choisi parmi les émulsifiants anioniques et non ioniques, et
- de l'eau.

13. Dispersion de polymère selon la revendication 11, contenant un polymère qui contient sous forme copolymérisée, comme uniques monomères,

- 43 à 47% en poids, par rapport au poids total des monomères utilisés pour la polymérisation, de méthacrylate de N,N-diéthylaminoéthyle a), et
- 53 à 57% en poids, par rapport au poids total des monomères utilisés pour la polymérisation, d'au moins un composé b).

14. Agent de revêtement, contenant une dispersion aqueuse de polymère Pd), telle que définie dans l'une quelconque des revendications 11 à 13 ou une composition polymère pouvant être obtenue à partir de celle-ci par séchage.

15. Agent pharmaceutique ou aliment, contenant

A) une composition polymère pouvant être obtenue par séchage et/ou filmage d'une dispersion de polymère telle que définie dans l'une quelconque des revendications 11 à 13,
B) au moins une substance active pharmaceutiquement acceptable ou un complément alimentaire et
C) le cas échéant au moins un adjuvant acceptable pharmaceutiquement ou dans le domaine alimentaire.

16. Agent pharmaceutique ou aliment selon la revendication 15 sous forme d'une forme d'administration orale, comprenant un revêtement à base d'une dispersion aqueuse de polymère Pd), telle que définie dans l'une quelconque des revendications 1 à 10, ou d'une composition polymère pouvant être obtenue à partir de celle-ci par séchage.

17. Utilisation d'une dispersion aqueuse de polymère Pd), telle que définie dans l'une quelconque des revendications 11 à 13, pour la fabrication de membranes, dans la cosmétique, dans la phytoprotection, pour le revêtement de semences, dans des aliments, dans les médicaments vétérinaires, dans l'alimentation animale, comme matière première pour adhésifs, pour la fabrication du papier, comme liant ou adjuvant pour les cuirs et les textiles, comme revêtement de surface microbicide, dans le domaine des non-tissés, dans les agents de lavage et de nettoyage, pour la fabrication d'enduits, dans le secteur de la construction.

18. Utilisation d'une dispersion aqueuse de polymère Pd), telle que définie dans l'une quelconque des revendications 11 à 13, en tant qu'agent de revêtement ou dans des agents de revêtement pour les cosmétiques, les aliments, les médicaments vétérinaires, l'alimentation animale, les semences, l'industrie du textile, du papier, de l'impression, du cuir et des adhésifs.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE S1090381 A
- DE S1219175 A
- DE OS2135073 A
- DE S2512238 A
- DE 1090381
- DE 1219175
- DE 2135073
- DE OS2838278 A
- GB 1324087 A
- DE 3426587 A1

- DE 3049179 A1
- DE 2512238
- EP 0058765 A2
- WO 2005055986 A
- WO 2005056619 A
- WO 0005307 A
- WO 02067906 A
- WO 2004019918 A
- US 4269749 A
- EP 101007 B

**In der Beschreibung aufgeführte Nicht-Patentliteratur**

- **K. C. BERGER ; G. BRANDRUP.** Polymer Handbook. John Wiley & Sons, 1989, II/81-II/141
- Methoden der organischen Chemie. **HOUBEN-WEYL.** Makromolekulare Stoffe. Georg-Thieme-Verlag, 1961, vol. X!V/1, 411-420
- Methoden der organischen Chemie. **HOUBEN-WEYL.** Makromolekulare Stoffe. Georg-Thieme-Verlag, 1961, 192-208
- **KARL HEINZ WALLHÄUßER.** Praxis der Sterilisation, Desinfektion - Konservierung. Georg Thieme Verlag, 1995, 465-652

- *ZFL-Zeitschrift für Lebensmitteltechnologie und -Verfahrenstechnik,* 1982, vol. 33 (3), 139 ff
- **FIEDLER, H. P.** Lexikon der Hilfsstoffe für Pharmazie, Kosmetik und angrenzende Gebiete. ECV-Editio-Cantor-Verlag, 1996
- *Cosmetics and Toiletries,* 1996, vol. 111 (8), 73ff